# EUROPEAN PATENT APPLICATION

(11) **EP 4 159 247 A1**
(43) Date of publication of application: **05.04.2023**
(21) Application number: 22198586.4
(22) Date of filing: 29.09.2022
(51) Int. Cl.: A61L 24/00, A61K 35/32, A61L 27/36

(54) **MODIFIED GRAFTS**

(30) Priority: 29.09.2021 US 202163249639 P
(71) Applicant: Musculoskeletal Transplant Foundation, Edison, New Jersey 08837 (US)
(72) Inventor: BASILE, Michael Vincent, Colts Neck NJ, 07722 (US); BHATTACHARYA, Subhabrata, Metuchen NJ, 08840 (US); MCALLISTER, Michele Christine, Brick NJ, 08724 (US)
(74) Representative: Trinks, Ole

(57) **Abstract**

Grafts modified with one or more bioactive substances are provided, as well as methods to make and use them. More particularly, the present invention relates to modified grafts having characteristics which facilitate tissue generation, repair, and reconstruction, and which are modified with bioactive substances, such as one or more proteins and minerals, whose bioactivity further facilitates tissue generation, repair, and reconstruction. Methods for producing the modified grafts include depositing the one or more bioactive substances onto, into, or both, a substrate material. In certain exemplary embodiments, the substrate material comprises a tissue derived matrix produced by processing one or more tissue samples, and the bioactive materials are precipitated from a solution produced during that processing, such as during demineralization of bone tissue samples or delipidation of adipose tissue samples, wherein the one or more bioactive substances comprise proteins and minerals endogenous to bone or adipose tissue, respectively.

## Description

### CROSS-REFERENCE TO RELATED APPLICATIONS

The present application claims the benefit of U.S. Provisional Application No. 63/249,639, filed September 29, 2021, the entire disclosure of which is incorporated by reference herein.

### FIELD

The present invention relates generally to grafts modified with one or more bioactive substances. More particularly, the present invention relates to modified grafts having characteristics which facilitate tissue generation, repair, and reconstruction, and which are modified with bioactive substances, such as one or more proteins and minerals, whose bioactivity further facilitates tissue generation, repair, and reconstruction.

### BACKGROUND

Grafts are prevalent in medicine today for treating many conditions such as wounds or other damage to tissue, or to treat or modify undesirable aesthetic characteristics of tissue or a body feature, regardless of cause or origin. Such treatments may involve intentionally causing or facilitating a physiological effect at the site tissue or a body feature of a recipient that is to be treated. Such physiological effects include, but are not limited to, one or more of inducing, participating in, facilitating, or otherwise enhancing biological processes such as, without limitation, healing, generating, regenerating, repairing and reconstructing tissue or a body feature, affected by a condition being treated.

There are many natural materials, synthetic materials, and combinations of natural and synthetic materials, which are known and used to produce grafts having desired characteristics. The preferred graft characteristics typically depend on the type of tissue or body feature and the condition thereof to be treated, as well as the nature of biological processes intended to be affected by the selected treatment.

Several bioactive substances including, but not limited to, proteins, lipids, minerals, organic and inorganic ions, cells, cells components, and inorganic substances, are known to, or are suspected of, causing or facilitating physiological effects which are supportive of one or more of the foresaid biological processes. These substances may be derived from natural biological sources, such as living animals, plants, or microorganisms, as well as from tissue samples which have been recovered from one or more living or cadaveric donors. Bioactive substances useful for inclusion with grafts may also be obtained from synthetic processes or sources.

Some grafts include a substrate which is formed from natural or synthetic materials, or a combination thereof, and which is intended to provide a scaffold for the infiltration, migration, and residence of cells and other bioactive substances at a treatment site at which a graft has been implanted. Sometimes one or more components of the substrate itself (e.g., collagens, glycoproteins, proteoglycans, natural and synthetic bioabsorbable polymers, etc.), will act as bioactive substances and induce, facilitate, or both induce and facilitate, one or more biological processes at the treatment site by which new tissue is generated to repair, reconstruct, or otherwise modify tissue or the body feature undergoing treatment.

In other cases, one or more bioactive substances (e.g., proteins, lipids, minerals, cells, etc.) are provided to the treatment site in any of several ways including, but not limited to, being recruited or attracted to the treatment site from the recipient's healthy or nearby tissues, being provided in combination with the graft, or even being provided to the treatment site separately from the graft. Bioactive materials provided with the graft may, for example without limitation, be one or more bioactive substances which are endogenous to and resident in the scaffold material (such as the bone tissue derived matrices already mentioned). Alternatively, or in addition, bioactive substances may be added to or combined with the scaffold material during or after producing the graft from the scaffold material.

U.S. Patent No. 9,962,467 describes grafts comprising demineralized bone matrices which retained some amount of one or more bioactive substances (e.g., growth factors and calcium) which were endogenous to bone tissue samples, even after the bone tissue samples were demineralized and subjected to other processing techniques to produce the matrices. Thus, the grafts of U.S. Patent No. 9,962,467 which contained those demineralized bone matrices also contained growth factors, such as bone morphogenetic proteins (BMPs), vascular endothelial growth factors (VEGFs), and platelet derived growth factors (PDGFs), which facilitated the repair of bone tissue and the generation of new bone tissue at treatment sites.

U.S. Patent No. 6,911,212 describes addition of several bioactive substances to a scaffold material comprising demineralized bone matrix (DBM) powder to produce a bone treatment graft. The DBM powder was mixed and combined with a solution of sodium hyaluronate in phosphate buffered saline to produce a bone repair graft that stimulates and facilitates generation of new bone tissue. U.S. Patent No. 6,911,212 acknowledges the benefits of adding bioactive substances such as BMP growth factors to such bone repair grafts, although it is silent on the source of the BMPs.

Many techniques have been developed for adding or combining additional bioactive substances grafts or substrates to produce grafts which don't already contain the desired bioactive substances or don't contain the desired amount or concentration of bioactive substances. Combining techniques include, but are not limited to, mixing, soaking, blending, drying, and combinations of these and other techniques. As discussed above, the bone repair grafts described in U.S. Patent No. 6,911,212 are produced by simply mixing DBM powder with bioactive substances or mixtures or solutions containing them to produce a generally homogenous putty material suitable for implanting in a bone defect or void.

By comparison, U.S. Patent Application Publication No. 2021/0178021 (US20210178021) describes a bone graft composition comprising demineralized bone particles which are "infused" with a supernatant of biologic material derived from bone marrow, or a polyampholyte cryoprotectant, or both. The supernatant of biologic material of US20210178021 comprises biologically active and inactive cell components such as cell fragments, cellular excretions (e.g., osteoinductive growth factors), cellular derivatives, and extracellular components and, optionally, also whole cells. According to US20210178021, the demineralized bone particles are combined with bioactive substances and other constituents of the supernatant of biological material by infusing, which is accomplished by exposing demineralized bone tissue particles and the supernatant to either a negative pressure (e.g., a vacuum) which draws the supernatant into the bone particles, or positive pressure to drive the supernatant into the bone. Infusing may be followed by drying the infused bone particles, such as by lyophilization.

In a preferred embodiment described in US20210178021, demineralized bone particles are combined with the supernatant comprising bioactive, as well as inactive, substances and a polyampholyte cryoprotectant which are all together exposed to either negative or positive pressure to draw or drive the supernatant and polyampholyte cryoprotectant into the bone particles, followed by lyophilization which causes the cryoprotectant to form a coating externally enveloping each of the bone particles along with the constituents of the supernatant. Thus, the bone graft composition comprises a bone tissue derived matrix with bioactive and other substances distributed and freeze-dried onto and within the matrix, as well as optionally coated and enveloped by polyampholyte cryoprotectant which is also freeze-dried thereon.

Grafts comprising a substrate and one or more bioactive substances which have been combined by techniques other than simply mixing or blending them together, or depositing and drying bioactive substances onto a substrate could provide improved grafts capable of further inducing and facilitating biological processes related to healing, generating, regenerating, repairing and reconstructing tissue affected by a condition to be treated. Such grafts and methods for making and using them are provided hereinbelow.

### SUMMARY

The present invention relates generally to grafts modified with one or more bioactive substances and having characteristics which facilitate healing, generating, regenerating, repairing and reconstructing tissue or a body feature, affected by a condition to be treated using the graft. The modified grafts described and contemplated herein comprise a substrate material and one or more bioactive substances precipitated thereon, therein, or both, from a solution which was contacted with the substrate.

The substrate material of the grafts may be derived or obtained from natural material, synthetic material, or a combination thereof. In some embodiments, the substrate material comprises a tissue derived matrix produced by processing one or more first tissue samples, each of which was recovered from one or more donors.

The bioactive substances comprise one or more proteins, lipids, minerals, organic ions, inorganic ions, cells, cells components, and inorganic substances such as minerals. Any of the bioactive substances may, independently of the others, be derived or obtained from one or more natural sources, natural processes, synthetic sources, synthetic processes, or combinations thereof. In some embodiments, one or more bioactive substances may be endogenous to and derived or obtained from one or more second tissue samples, each of which was recovered from one or more donors. Each of the one or more first tissue samples may be the same or different tissue types as each of the one or more second tissue samples. Furthermore, the one or more first tissue samples may be the same as the one or more second tissue samples.

An exemplary method for producing a modified graft comprising a substrate material and one or more bioactive substances deposited thereon comprises the steps of: providing a substrate material, providing a solution containing one or more bioactive substances, contacting the substrate material and the solution, depositing the one or more bioactive substances on the substrate material, in the substrate material, or both, by precipitating at least one of the one or more bioactive substances from the solution while in contact with at least a portion of the substrate material. The method may further comprise drying the substrate material and one or more bioactive substances precipitated thereon to produce the modified graft.

In some embodiments, the step of providing a substrate material comprises processing one or more first tissue samples, each of which was recovered from one or more donors. Processing the one or more first tissue samples may comprise one or more physical techniques, one or more chemical techniques, or a combination thereof.

In some embodiments, the step of providing a solution containing one or more bioactive substances comprises processing one or more second tissue samples, each of which was recovered from one or more donors. Processing the one or more first tissue samples may comprise one or more physical techniques, one or more chemical techniques, or a combination thereof, wherein the one or more bioactive substances comprise at least one bioactive substance which is endogenous to at least one of the one or more second tissue samples. In some embodiments, processing one or more second tissue samples to provide the solution containing the one or more bioactive substances comprises extracting the one or more bioactive substances from the one or more second tissue samples into a solvent. The solvent may be one or more biocompatible solvents such as water, saline, buffered saline, and combinations thereof.

### BRIEF DESCRIPTION OF THE FIGURES

The present invention will be further explained with reference to the attached drawings, wherein like structures are referred to by like numerals and/or letters throughout the several views. The drawings shown are not necessarily to scale, with emphasis instead generally being placed upon illustrating the principles of the present invention.
FIGS. 1A-1D are results of scanning electron microscopy (SEM) performed on samples produced by the method of Example 1.1; and
FIGS. 2A-2C are results of x-ray diffraction analysis performed on samples produced by the method of Example 4.3.

### DETAILED DESCRIPTION

Detailed embodiments of the present invention are disclosed herein. It should be understood that the disclosed embodiments are merely illustrative of the invention which may be embodied in various forms. In addition, each of the examples given in connection with the various embodiments of the invention is intended to be illustrative, and not restrictive. Further, the figures are not necessarily to scale, and some features may be exaggerated to show details of particular components. In addition, any measurements, specifications and the like shown in the figures are intended to be illustrative, and not restrictive. Therefore, specific structural and functional details disclosed herein are not to be interpreted as limiting, but merely as examples for teaching one skilled in the art to variously employ the present invention.

Modified grafts described and contemplated herein comprise; a substrate material which is formed from natural or synthetic materials, or a combination thereof; and one or more bioactive substances deposited onto at least a portion of the substrate material, into at least a portion of the substrate material, or both, by precipitating at least one of the one or more bioactive substances out of a solution in contact with the substrate material.

The substrate material provides a scaffold for the infiltration, migration, and residence of cells and other bioactive substances at a treatment site at which a graft has been implanted. In some embodiments, one or more components of the substrate itself (e.g., collagens, glycoproteins, proteoglycans, natural and synthetic bioabsorbable polymers, etc.), are bioactive substances and will induce, facilitate, or both induce and facilitate, one or more biological processes at the treatment site by which new tissue is generated to repair, reconstruct, or otherwise modify tissue or the body feature undergoing treatment.

Like conventional grafts, the modified grafts described and contemplated herein are useful for treating many conditions such as wounds or other damage to tissue, or to treat or modify undesirable aesthetic characteristics of tissue or a body feature, regardless of cause or origin. Such treatments may involve intentionally causing or facilitating a physiological effect at the site tissue or a body feature of a recipient that is to be treated. Such physiological effects include, but are not limited to, one or more of inducing, participating in, facilitating, or otherwise enhancing biological processes such as, without limitation, healing, generating, regenerating, repairing and reconstructing tissue or a body feature, affected by a condition being treated. Additionally, modified grafts in accordance with the present description provide further enablement and enhancement to the aforesaid biological processes which are helpful in treating damaged and diseased tissue, as well as aesthetically undesirable body features.

The materials suitable for producing substrate material include natural materials, synthetic materials, and combinations of natural and synthetic materials, and are selected to produce grafts having desired characteristics depending on several factors including, without limitation, the type of tissue or body feature and the condition thereof to be treated, as well as the nature of biological processes intended to be affected by the selected treatment. For example, without limitation, bone tissue samples recovered from one or more donors may be processed by physical and chemical techniques to provide natural, tissue derived matrices which, because of the collagen composition of bone tissue samples and other constituents and endogenous bioactive substances contained therein, are particularly useful for producing grafts effective to treat bone defects, injuries, voids, etc.

Several bioactive substances including, but not limited to, proteins, minerals, organic and inorganic ions, cells, cells components, and inorganic substances, are known to, or are suspected of, causing or facilitating physiological effects which are supportive of one or more of the foresaid biological processes. These substances may be derived from natural biological sources, such as living animals, plants, or microorganisms, as well as from tissue samples which have been recovered from one or more living or cadaveric donors. Bioactive substances useful for inclusion with grafts may also be obtained from synthetic processes or sources.

### DEFINITIONS

The following definitions are provided for clarification and enhancing understanding of the following description, but are not intended to be limiting.

As used herein, the term "bioactive" means a substance or material which has a physiological effect on a recipient of the bioactive substance, where the physiological effect may be an intended component of treating of a condition. Physiological effects of bioactive substances comprise, but are not limited to, one or more of inducing, participating in, facilitating, or otherwise enhancing biological processes such as, without limitation, healing, generating, regenerating, repairing and reconstructing tissue, an organ, or a body feature, affected by a condition to be treated. Bioactive substances may be administered to a recipient in any suitable and effective manner, whether directly or indirectly to the tissue, organ, or body feature to be treated. It is common for one or more bioactive substance to be administered to a recipient separately (alone) or in combination with other substances, materials or devices.

The term "biocompatible," as used here, means that a graft or implant, when implanted in a subject, does not cause one or more adverse effects such as, without limitation, toxicity, injury, foreign body reaction or rejection, irritation, allergic or other histamine reaction, disruption of cellular structure or function, etc.

As used herein, the term "condition" refers to a variety of healthy and unhealthy states of a living subject and its organs, tissues, and biological systems, and includes both normal, healthy and functioning states, as well as abnormal, unhealthy, damaged, and malfunctioning states, whether caused by a disorder, disease, infection, injury, trauma, or other mechanism or event.

The term "contacting" and "implanting" and their various grammatical forms, are used interchangeably herein when discussing or explaining the placement of a graft and refer to a state or condition of touching, or of immediate or local proximity, internally or externally, such as positioning or implanting a substance, composition, graft, or other device, in, on, or proximate to, tissue, an organ, or a body feature to be treated. Contacting a substance or composition to a target destination, such as a body feature or an implant site, may occur by any means of administration known to persons of ordinary skill in the relevant art.

The terms "derived," "derived from," and "produced from" and all their grammatical forms, are used herein interchangeably to mean that the material or composition being described is produced by subjecting one or more specified starting materials to one or more process steps or techniques, which may be physical, chemical, or combinations of physical and chemical techniques, as are appropriate depending on the starting material and intended material or composition product. For example, without limitation, a "tissue derived matrix" is a composition which has been produced by subjecting a selected tissue sample to one or more process steps or techniques, which may be physical, chemical, or combinations thereof. (e.g., recovering the tissue sample from a donor, cutting to size, disinfecting, decellularizing, and drying by lyophilizing). A component of a graft which is produced from a biocompatible synthetic polymer means that the component is produced by subjecting the selected biocompatible synthetic polymer to one or more process steps or techniques, which may be physical, chemical, or combinations thereof (e.g., optionally blending, combining, mixing, etc., the selected polymer with one or more additives such as rheology modifiers, emulsifiers, dispersing agents, stabilizers, etc., and shaping by thermoforming, extrusion, crosslinking, etc.).

As used herein, the term "donor" means a living or deceased mammal from which one or more tissues, organs, other body parts, or portions thereof, are recovered or harvested. Suitable mammalian donors include, but are not limited to: human, primate, bovine, porcine, equine, ovine, rodent, leporine, canine, feline, etc.

As used herein, the term "endogenous" means produced, synthesized, or otherwise originating by, from, or within a cell, tissue, or organ.

As used herein, the term "graft" means a biologically compatible material, tissue, or substance which is introduced into the body of a subject, either permanently or temporarily, to replace, improve or supplement the structure or function of tissue, an organ, or other body feature, of a subject or patient. A graft may be used for the administration or delivery of a therapeutic agent or substance. Grafts may be absorbed or integrated, in whole or in part, into a patient's body after implantation.

As used herein, the terms "implant" and "implanting," in all their grammatical forms, mean the placement of a graft, or other substance or device, in, or, or proximate to tissue, an organ or body feature of a recipient that is undergoing treatment using the graft or other substance or device. The term "implantation site," as used herein, means the portion or region of a recipient at which either: (1) a graft or other substance or device has been implanted, (2) one or more tissues, organs or body features, which are undergoing treatment are located, or (3) both of the foregoing.

As used herein, the terms "recipient," "host," "subject," and "patient" are used interchangeably to mean a living organism receiving treatment for a condition present in one or more of its tissue, organs, and body features, where the treatment may, without limitation, include one or more of: performing a surgical procedure, delivering, providing, or implanting, a graft, an implant, pharmaceutical agents, antimicrobial (e.g., antibiotic, antibacterial, antifungal, antiprotozoal, antiparasitic, etc.) agents, and the like. A recipient may, but is not required to, be a human. A recipient may also be any other living animal, including without limitation, mammals other than human, birds, reptiles, amphibians, etc.

As used herein, the term "tissue derived matrix" means a material or substance which is produced, by one or more processing steps and techniques, from one or more samples of tissue recovered from one or more donors. The one or more processing steps and techniques may be physical, chemical, and combinations thereof. The samples of tissue may be the same or different types of tissue as one another. Furthermore, it is possible for a single tissue sample to include more than one type of tissue when recovered and may or may not be subjected to separation of the different types of tissue during processing. The donor of one or more of the tissue samples from which a tissue derived matrix is produced may be: the same individual as the recipient of a graft comprising the tissue derived matrix (i.e., autogenic), or a different individual of the same species as the recipient (i.e., allogenic), or a different species as the recipient (i.e., xenogenic).

The term "treatment" and all its grammatical forms, as used herein, mean the use of an agent, procedure, or regimen, such as a pharmaceutical agent, surgical procedure, implant, prosthetic device, diet modification, exercise, etc., in an attempt to cure or mitigate a disease, injury, or other undesired condition, whether medically necessary or aesthetically preferred. Accordingly, tissue, organs, or body features of a recipient, may be subjected to "treatment," which may involve implanting one or more grafts, to generate, regenerate, repair, or reconstruct damaged or malfunctioning tissue, organs, or body features, or for cosmetic or aesthetic reasons,

### MODIFIED GRAFTS

Grafts modified with one or more bioactive substances and having characteristics which facilitate healing, generating, regenerating, repairing and reconstructing tissue or a body feature, affected by a condition to be treated using the modified graft are described hereinbelow. The modified grafts generally comprise a substrate material and one or more bioactive substances deposited thereon, therein, or both, by precipitating the bioactive substances from a solution which is in contact with the substrate. Precipitating the one or more bioactive substances may be performed under negative pressure, positive pressure, or neither (i.e., under approximately atmospheric pressure).

One or more of the precipitated bioactive materials, either individually or as coprecipitated compounds, may or may not be bonded to the substrate material, such as by covalent bonds, ionic bonds, other physiochemical bonds (e.g., van der Waals forces, etc.), and combinations thereof. At least a portion of such bonds may be stronger than are found in grafts on which substances have been deposited by non-precipitation techniques such as spraying, coating, or soaking with solution followed by condensation, air or heat drying, etc.

The substrate material of the grafts may be derived or obtained from natural material, synthetic material, metals and their alloys, or a combination thereof. The substrate material may have any of many suitable characteristics, as desired based on the intended use of the graft, including without limitation, being soft or not, being porous or not, being flexible or not, being elastic or not, being impervious or not, being rigid or not, and the like. The substrate material may also be in any suitable physical form, as desired based on the intended use of the graft, including without limitation, particulate, powder, fibers (e.g., elongate particles or pieces), homogenous in size, shape or both, heterogenous in size, shape, or both, multi-phase (e.g., a combination of one or more of a liquid, a solid, a gel, a putty, etc.), pieces, an injectable flowable form, monolithic, multi-component, etc.

In some embodiments, the substrate material comprises a tissue derived matrix produced by processing one or more first tissue samples which were recovered from one or more donors. Each of the one or more first tissue samples may have been processed by performing one or more physical techniques, one or more chemical techniques, or combinations thereof. In some embodiments, the tissue derived matrix comprises particles. In some embodiments, the tissue derived matrix comprises a putty. In some embodiments, the tissue derived matrix comprises one or more three dimensional pieces, such as without limitation, a single-piece bone spacer, one or more processed bone blocks, or even a multi-component bone prosthesis. In some embodiments, the tissue derived matrix comprises at least partially demineralized bone tissue (cortical, cancellous, or both), non-demineralized bone tissue (cortical, cancellous, or both), or combinations thereof.

In some embodiments, the substrate material comprises other than a tissue derived matrix, such as at least one natural biocompatible material, polymer or copolymer (e.g., collagen, saccharide, gelatin, polysaccharide, starch, lignan, cellulose, silk, tricalcium phosphate, etc.). In some embodiments, the substrate material comprises other than a tissue derived matrix, such as, at least one synthetic material, polymer, or copolymer (e.g., ceramics, bioactive glass (including silicon dioxide, calcium oxide, sodium oxide, etc.), polyglycolide, polyester amides, polyanhydrides, polycaprolactone, polyurethanes, etc.). In some embodiments, the substrate material comprises other than a tissue derived matrix, such as at least one metal (e.g., titanium, stainless steel, etc.), at least one metal alloy (e.g., cobalt-chromium alloy, etc.), or combinations thereof.

The bioactive substances comprise one or more proteins, minerals, organic ions, inorganic ions, cells, cells components, and inorganic substances. Any of the bioactive substances may, independently of the others, be derived or obtained from one or more natural sources, natural processes, synthetic sources, synthetic processes, or combinations thereof. In some embodiments, one or more bioactive substances may be derived or obtained from one or more second tissue samples which were recovered from one or more donors. In some embodiments, the one or more bioactive substances have been extracted from the one or more second tissue samples into solution. Furthermore, each of the one or more second tissue samples may have been processed by performing one or more physical techniques, one or more chemical techniques, or combinations thereof.

In some embodiments, the step of providing a solution containing the one or more bioactive substances may be performed by subjecting the second tissue samples to one or more processing steps. Accordingly, the solution containing one or more bioactive substances may be the direct or indirect product of one or more process steps such as, without limitation, cleaning, demineralizing, decellularizing, delipidizing, devitalizing, proteoglycan removal, elution, protein extraction, physical agitation, etc. In some embodiments, the step of providing the solution containing one or more bioactive substances may further comprise one or more further process steps including, without limitation, separating, sorting, quantifying, analyzing, characterizing (i.e., identifying), filtering, concentrating, dehydration, dilution, stabilizing, dissolving, and the like.

In one exemplary embodiment, wherein the substrate material comprises a tissue derived matrix produced by processing one or more first tissue samples and the one or more bioactive substances were derived or obtained from one or more second tissue samples, the one or more first tissue samples are the same tissue type as the one or more second tissue samples. In further exemplary embodiment, the one or more first tissue samples are the same as the one or more second tissue samples. The tissue types of the one or more first tissue samples and, independently, the tissue types of the one or more second tissue types are selected from: adipose, amnion, artery, bone, cartilage, chorion, colon, dental, dermal, duodenal, endothelial, epithelial, fascial, gastrointestinal, growth plate, intervertebral disc, intestinal mucosa, intestinal serosa, ligament, liver, lung, mammary, meniscal, muscle, nerve, ovarian, parenchymal organ, pericardial, periosteal, peritoneal, placental, skin, spleen, stomach, synovial, tendon, testes, umbilical cord, urological, vascular, vein, and combinations thereof.

In some embodiments, the substrate material comprises two or more tissue derived matrices which have been produced from tissue samples of different tissue types. In some embodiments, the substrate material comprises a tissue derived matrix which has been produced by processing a tissue sample which comprises more than one tissue type. In one exemplary embodiment, the substrate material may comprise an osteochondral graft which includes both a bone tissue derived matrix and a cartilage tissue derived matrix. In another exemplary embodiment, the substrate material may comprise a bone-tendon entheses graft which includes both a bone tissue derived matrix and a tendon tissue derived matrix.

Different tissue types contain different types of cells, and different types of cells express or secrete different combinations and proportions of proteins (e.g., growth factors, cytokines, interleukins, proteoglycans, etc.), which in turn will induce, facilitate or cause formation (e.g., generation) of new tissue of different tissue types. It is further contemplated that in some embodiments, a modified graft may comprise a substrate material comprising a tissue derived matrix of a first tissue type and one or more bioactive substances deposited thereon, wherein the one or more bioactive substances are capable of inducing, facilitating or causing formation of tissue of a second tissue type which is different from the first tissue type. For example, without limitation, in an exemplary embodiment, a modified graft comprising a substrate material which comprises cartilage tissue derived matrix, may comprise bioactive substances derived from bone tissue samples, whereby the bone derived bioactive substances are precipitated onto the cartilage tissue derived matrix and those bone derived bioactive substances induce, facilitate or cause formation of new bone tissue on the cartilage derived matrix substrate.

In some embodiments, one or more of the bioactive substances may be derived or obtained from one or more natural sources, natural processes, or a combination thereof. For example, without limitation and regardless of source or origin, the bioactive substances may comprise proteins such as collagen, lipids, saccharides (such as mono-, di- and polysaccharides), cytokines, interleukins, cell binding peptides, and the like, and combinations thereof, which have or have not been derived from tissue samples recovered from one or more donors. Of course, the one or more bioactive substances may comprise proteins derived from a combination of tissue samples and natural sources other than tissue samples.

In some embodiments, one or more of the bioactive substances comprises one or more proteins or other substances may, independently of the others, be derived or obtained from one or more synthetic sources, synthetic processes, or a combination thereof. For example, without limitation, the bioactive substances may comprise a recombinant protein (e.g., rhMBP2, a recombinant peptide, a synthetic peptide, a synthetic sequence, a synthetic cytokine, etc.

### METHODS FOR PRODUCING MODIFIED GRAFTS

An exemplary method for producing a modified graft comprising a substrate material and one or more bioactive substances deposited thereon comprises the steps of: providing a substrate material, providing a solution containing one or more bioactive substances, contacting the substrate material and the solution, depositing the one or more bioactive substances on the substrate material, in the substrate material, or both. In some embodiments, the depositing step is performed by precipitating or co-precipitating at least one of the one or more bioactive substances from the solution while in contact with at least a portion of the substrate material. The method may further comprise drying the substrate material and one or more bioactive substances precipitated thereon to produce the modified graft. Drying the substrate material and one or more bioactive substances precipitated thereon may, for example without limitation, be performed by air drying, heat drying, evaporation, sublimation, lyophilizing, and combinations thereof.

In some embodiments, the step of providing a substrate material comprises processing one or more first tissue samples, each of which was recovered from one or more donors. Processing the one or more first tissue samples may comprise one or more physical techniques, one or more chemical techniques, or a combination thereof.

In some embodiments, the step of providing a solution containing one or more bioactive substances comprises processing one or more second tissue samples, each of which was recovered from one or more donors. Processing the one or more first tissue samples may comprise one or more physical techniques, one or more chemical techniques, or a combination thereof, wherein the one or more bioactive substances comprise at least one bioactive substance which is endogenous to at least one of the one or more second tissue samples. In some embodiments, processing one or more second tissue samples to provide the solution containing the one or more bioactive substances comprises extracting the one or more bioactive substances from the one or more second tissue samples into a solvent. The solvent may, for example without limitation, be one or more biocompatible solvents selected from: water, saline, buffered saline, and combinations thereof.

The one or more first tissue samples may, but do not have to be, the tissue type as the one or more second tissue samples. For example, without limitation, where the one or more first tissue samples from which the substrate material is produced are bone tissue, the one or more second tissue samples from which the one or more bioactive substances are derived or produced are bone tissue. Furthermore, the one or more first bone tissue samples may, but do not have to, be the same tissue samples or portions thereof as the one or more second bone tissue samples.

As previously explained above, the step of providing a solution containing one or more bioactive substances, where the bioactive substances are derived from one or more tissue samples, may be performed using one or more process steps such as, without limitation, cleaning, demineralizing, decellularizing, delipidizing, devitalizing, proteoglycan removal, elution, protein extraction, physical agitation, etc. Furthermore, the step of providing the solution containing one or more bioactive substances may further comprise one or more further process steps including, without limitation, separating, sorting, quantifying, analyzing, characterizing (i.e., identifying), filtering, concentrating, dehydration, dilution, stabilizing, dissolving, and the like.

In some embodiments, the step of precipitating comprises inducing (or causing) precipitation, co-precipitation, or both, of at least one of the one or more bioactive substances from the solution comprises by manipulating one or more parameters (i.e., conditions, constituents, characteristics, etc.) of the solution. Manipulation of one or more parameters may comprises one or more adjustment techniques selected from: (a) adjusting the pH of the solution (e.g., by addition of sodium hydroxide or other biocompatible base); (b) adjusting concentration of one or more solvents in the solution (e.g., adding a C₁ - C₄ alcohol (e.g., ethanol)); (c) adding a buffered saline solution having a pH greater than the solution containing one or more bioactive substances; or (d) adjusting concentration of one or more salts in the solution (e.g., addition of a sodium or potassium salt). Additionally, as will be understood and practicable by persons of ordinary skill in the relevant art, the selection of which adjustment technique is performed and the degree of adjustment of a selected parameter can determine and enable control of which particular bioactive substance or substances (e.g., proteins, lipids, minerals, ions, cells, etc.) is precipitated or coprecipitated.

It is further noted that, in some embodiments, the step of precipitating may cause one or more bioactive substances to coprecipitate with one or more other bioactive substances. For example, without limitation, the step of precipitating may cause one or more proteins to coprecipitate with one another, one or more ions, or both. For example, without limitation, the step of precipitating may cause one or more minerals to coprecipitate with one another. For example, without limitation, the step of precipitating may cause one or more proteins to coprecipitate with one or more minerals, ions, lipids, or combinations thereof.

In some embodiments, the method for producing a modified graft further comprises the step of adding one or more additional precipitate components to the solution. At least a portion of the one or more additional precipitate components will be precipitated or co-precipitated onto the substrate material, with or without one or more of the bioactive substances, during the precipitating step. Additional precipitate components may be bioactive or not. Additional precipitate components include, without limitation, one or more of: pharmaceutical agents, antibodies, lubricants, excipients, radiopaque compounds, contrast agents, as well as other beneficial or useful substances, and combinations thereof.

Pharmaceutical agents suitable for use as additional precipitate components are not particularly limited and include, without limitation: broad spectrum, specific, and other antibiotics (e.g., tetracycline, penicillin, amoxicillin, gentamicin, etc.), analgesics (e.g., acetaminophen, naltrexone, etc.), steroidal and non-steroidal anti-inflammatories (e.g., corticosteroids, ibuprofen, naproxen aspirin, etc.), and combinations thereof. Embodiments of the modified graft in which an additional precipitate component comprising a pharmaceutical agent which includes one or more antibiotics, could, for instance, be useful to provide prophylactic action to prevent or minimize the risk of the patient developing infection post-implantation, such as from exposure to infectious agents in a hospital or other setting.

Inclusion and precipitation of one or more radiopaque compounds or contrast agents on the substrate material are intended to make at least a portion of the modified graft opaque to radiation (i.e., visible in x-ray photographs and under fluoroscopy). Other than being biocompatible, radiopaque compounds and contrast agents suitable for use as additional precipitate components are not particularly limited and include, for example without limitation, one or more of: iodine based contrasting agents, bismuth subcarbonate, barium sulfate, etc. Embodiments of the modified graft in which an additional precipitate component comprising a radiopaque agent, would, for instance, be useful to enable or facilitate position, identification, and orientation of the graft prior to, during, and/or after implantation, via open as well as minimally invasive procedures.

Any of the physical techniques and chemical techniques for processing tissue samples may, without limitation, be selected from size modification; cleaning; rinsing; separating and removing unwanted debris, material, tissue types, etc.; decellularizing; delipidizing; demineralizing; extracting; disinfecting; sterilizing; dehydrating (drying); crosslinking; treating, contacting, or combining with one or more of rheology modifiers, stabilizing agents, preservatives, and cryoprotectant agents.

While the aforesaid grafts and methods for making and using them will be described in detail hereinafter as applied to modified grafts comprising a substrate derived from donor tissue samples comprising bone tissue, and having endogenous bioactive proteins and minerals derived from the same donor tissue samples deposited thereon, it is not limited to such embodiments. Rather, persons of ordinary skill will recognize that the modified grafts may comprise substrate material derived from other than tissue samples, including other natural materials, synthetic materials, metals and their alloys, and combinations thereof, while the bioactive materials precipitated onto the substrate material need not be derived from the same tissue samples as are used to produce the substrate material, and need not be derived from tissue samples at all.

In an exemplary embodiment, the modified grafts comprise a substrate material derived from one or more bone tissue samples, with one or more bioactive substances precipitated thereon, therein, or both, wherein the one or more bioactive substances comprise at least proteins and minerals which are endogenous to the one or more bone samples and were contained and precipitated from a residual demineralizing solution produced during demineralizing at least one of the one or more bone tissue samples used to produce the substrate material. More particularly, such modified grafts can be understood as comprising three broad components, as follows.

Substrate Material: A variety of materials, both natural and synthetic, can be leveraged for this modified graft technology. This includes, without limitation, demineralized cortical bone fibers, cortical or cancellous particulates alone or mixed, cortical or cancellous blocks or cortical-cancellous blocks, chips, ceramic particulates, ceramic blocks, synthetic biodegradable and nonbiodegradable structures, synthetic biodegradable and nonbiodegradable particulates. The bone tissue samples may be fresh harvested tissue which has never been frozen. Besides bone, the substrate material may comprise another tissue type such as, without limitation, nerve, tendon, dermal, adipose, amnion, artery, cartilage, chorion, colon, dental, duodenal, endothelial, epithelial, fascial, gastrointestinal, growth plate, intervertebral disc, intestinal mucosa, intestinal serosa, ligament, liver, lung, mammary, meniscal, muscle, ovarian, parenchymal organ, pericardial, periosteal, peritoneal, placental, skin, spleen, stomach, synovial, testes, umbilical cord, urological, vascular, vein, and combinations thereof.

Recaptured/precipitated protein content: The precipitated active substance proteins may be of natural origin, such as originating from and being endogenous to the bone tissue samples from which the substrate material is produced. In such cases, when the bone tissue samples are demineralized to produce the substrate material, the active substance proteins (and minerals) remain in the resulting residual demineralizing solution (RDS). Such bioactive substance proteins may also be of a different origin also. For example, they can be obtained from natural extracted and/or purified proteins from other tissue sources including bone, blood, plasma etc. They can also be recombinant proteins of synthetic variety too. The proteins can be bone forming proteins, angiogenic proteins, cytokines and any other type of proteins. In case of bones, nerves, tendons, skin, etc., relevant bioactive substance proteins can be precipitated from a natural or synthetic origin.

Coprecipitated mineral bioactive substances /bioinorganics content: Inorganic mineral, which may also be contained in the RDS produced during demineralizing bone tissue to produce the substrate material, may be precipitated from the RDS onto, into, or both, the substrate material. Thus, the bioactive substances may include endogenous minerals of natural origin which have been separated and recovered from bone tissue samples and captured in the RDS, followed by deposition onto the bone tissue derived substrate material by precipitation out of the RDS. Alternatively, bioactive substances may include minerals of natural of synthetic origin. In case of synthetic origin, they can also be from a variety of sources including, without limitation, hydroxyapatites, doped hydroxyapatites and other materials.

Thus, in an exemplary embodiment, an advanced bone void filler (BVF+) graft (the modified graft "Composition") is provided, which is comprised of demineralized cortical bone (substrate material) that is recombined with minerals and proteins (bioactive substances) that are captured from the residual demineralizing solution, or RDS, (i.e., acid solution) by subjecting the RDS to a series of precipitation and purification steps. These modified constructs/grafts are then lyophilized and may be stored at room temperature. These modified constructs/grafts can also be maintained in other formats such as with a suitable media at room temperature or other temperatures.

The modified graft, i.e., an advanced bone void filler (BVF+) graft has multifaceted uses in different formats. These include use as a bone graft extender, bone graft substitute, and bone void filler in bony voids or gaps of the musculoskeletal system. BVF+ technology to precipitate proteins and/or growth factors on cortical spacers, cancellous blocks, cortical fibers, cancellous or cortical powders or particulates, partially or fully demineralized cortical blocks, or as a coating on or in any suitable graft. BVF+ technology can also be used to precipitate proteins and/or growth factors onto, into, or both, matrices derived from different tissue types such as tendons, synthetics, ceramics etc.

To demineralize bone tissue samples, they are typically treated with hydrochloric acid - but in addition to removing endogenous minerals (e.g., calcium) this demineralizing process also dislodges a significant portion of the bone's endogenous proteins (i.e., bioactive substances), as well as other bioactive substances mentioned above. The endogenous minerals and proteins are both eluted into the residual demineralization solution (RDS) during this step. Traditionally, in demineralized bone matrix (DBM) processing, this RDS has been discarded as waste material. In the present novel process, the pH of the RDS is altered to precipitate and recapture a portion of the dissolved minerals and proteins using the demineralized bone tissue as a substrate material. The recovered proteins and minerals precipitate on the demineralized bone fibers in a fine particulate, resulting in a more complete graft structure, i.e., one exemplary embodiment of modified grafts.

Bioactive substances such as the following proteins listed in Table 1 are endogenous to bone tissue and are depleted during demineralization.

**Table 1 - Some Proteins Endogenous to Bone Tissue**

| | | |
|---|---|---|
| BMP-2 | Bone morphogenetic protein 2 (BMP-2) (Bone morphogenetic protein 2A) (BMP-2A) | Role in bone formation, joint anti-inflammation and synovial repair. Recombinant BMP-2 may induce bone formation and osteoblastic differentiation by regulating endochondral ossification |
| BMP-6 | Bone morphogenetic protein 6 (BMP-6) (VG-1-related protein) (VG-1-R) (VGR-1) | Promotes and regulate bone development, growth, remodeling and repair |
| BMP-7 | Bone morphogenetic protein 7 (BMP-7) (Osteogenic protein 1) (OP-1) (Eptotermin alfa) | Induces bone formation and favourably influence the process of bone repair. The growth factor has been demonstrated to have the capacity to recruit and stimulate differentiation and proliferation of osteogenic cell populations. |
| Dkk-1 | Dickkopf-related protein 1 (Dickkopf-1) (Dkk-1) (hDkk-1) (SK) | A regulator of bone formation. |
| MMP-3 | Stromelysin-1 (SL-1) (EC 3.4.24.17) (Matrix metalloproteinase-3) (MMP-3) (Transin-1) | A matrix-bound proenzyme form, may act as a reservoir for later activation. Bone remodeling influenced by MMP-3; Increased proliferation and migration of MSCs |
| OPG | Tumor necrosis factor receptor superfamily member 11B (Osteoclastogenesis inhibitory factor) (Osteoprotegerin) | A critical regulator of bone resorption. OPG inhibits terminal differentiation and activation of osteoclasts |

Bone morphogenic proteins (BMPs) are a group of growth factors, also known as cytokines and metabologens. Originally discovered by their ability to induce the formation of bone and cartilage, BMPs are now considered to constitute a group of pivotal morphogenetic signals.

Cytokines are a broad and loose category of small proteins that are important in cell signaling. Cytokines are peptides which cannot cross the lipid bilayer of cells to enter the cytoplasm. Cytokines have been shown to be involved in autocrine signaling, paracrine signaling and endocrine signaling as immunomodulating agents.

Angiogenic proteins are growth factors that induce formation and development of new capillary blood vessels in living tissue. Angiogenic proteins include, for example, vascular endothelial growth factors (VEGF) (e.g., placental growth factor (PlGF)), platelet-derived growth factor (PDGF), fibroblast growth factor (FGF), epidermal growth factor (EGF), hepatocyte growth factor (HGF), and insulin-like growth factor (IGF). Angiogenesis is necessary so that a growing or enlarging tissue, with its increasing metabolic needs, obtains an adequate blood supply providing oxygen, nutrients and waste drainage.

Key new and improved features of the present modified grafts include:
^{∗} Elevated levels of osteoinductive proteins relative to traditional demineralized bone matrix (DBM) grafts
^{∗} Elevated levels of additional structural proteins and non-structural proteins relative to traditional demineralized bone matrix (DBM) graft
^{∗} Elevated levels of angiogenic proteins relative to traditional DBM grafts
^{∗} Elevated levels of bone mineral content/bioinorganics compared to traditional DBM grafts
^{∗} Low pH tissue-form which may preserve the proteins better and make the handling more facile especially in an MIS environment. (See, Bueno, E. M. and J. W. Ruberti (2008), "Optimizing Collagen Transport through Track-Etched Nanopores." J. Memb. Sci. 321(2): 250-263.)

The modified grafts described and contemplated herein provide several advantageous features and improvements over the prior art. For instance, elevated levels of proteins in these modified grafts promote more rapid and complete healing of bone, as well as more rapid and complete vascularization of bone.

Elevated levels of bone minerals in these modified grafts also promote more rapid and complete healing and vascularization of bone. (See, Buddhachat K, et al. (2016) Elemental Analysis of Bone, Teeth, Horn and Antler in Different Animal Species Using Non-Invasive Handheld X-Ray Fluorescence. PLoS One 11(5):e0155458.; and Dermience M, Lognay G, Mathieu F, & Goyens P (2015) Effects of thirty elements on bone metabolism. J Trace Elem Med Biol 32:86-106.) Reprecipitated nanocrystals of bone minerals may form focal epitopes for facile mineralization offering a low activation energy pathway for mineralization by the osteoblasts during new bone formation. (See, Dey A, et al. (2010) The role of prenucleation clusters in surface-induced calcium phosphate crystallization. Nat Mater 9(12): 1010-1014.)

Incorporation of essential and non-essential trace inorganic elements, which act as cofactors for different natural proteins, can be preserved providing better performance. (See, Wang W & Yeung KWK (2017) Bone grafts and biomaterials substitutes for bone defect repair: A review. Bioact Mater 2(4):224-247.) Elevated levels of endogenous bone mineral content promote sustained release of relevant proteins by co-precipitating proteins along with them to the DBM matrix.

Each of the precipitated minerals from RDS, including magnesium, strontium, silicon, zinc, copper, lithium, and cobalt, contribute to creating a bone-mimicking graft material. Reprecipitated endogenous bone minerals (which may sometimes referred to as "bioinorganics") are known to support osteogenesis and angiogenesis on their own, showing an osteostimulatory effect.

For instance, magnesium induces hypoxia-inducible factor (HIF) and activates peroxisome proliferator-activated receptor-gamma coactivator 1alpha (PGC-1a) production in undifferentiated and differentiated hBMSCs, respectively. This stimulates the production of VEGF. Magnesium enters dorsal root ganglion (DRG) neurons and promotes the release of calcitonin gene-related peptide (CGRP) and then stimulates the PDSCs to express the genes contributing to osteogenic differentiation.

Strontium promotes the activity of bone-forming osteoblastic cells, while inhibiting the bone resorbing osteoclasts. It activates CaSR and downstream signaling pathways. It increases the OPG production and decreases RANKL expression. This promotes osteoblast proliferation, differentiation, and viability and induces the apoptosis of osteoclasts that result to the decrease of bone resorption.

Silicon has been shown to induce angiogenesis by upregulating NOS leading to increased VEGF production at low concentration when cultured with human dermal fibroblasts. Osteogenic mechanism is not well understood. However, Si⁴⁺ at higher concentration has been shown to play a vital role in the mineralization process.

Zinc is found to get involved in the structural, catalytic or regulation of ALP expression in which it plays an important role in osteogenesis and mineralization. It is also believed that zinc is able to suppress the osteoclastic resorption process.

Copper is reported to be a hypoxia-mimicking factor leading to the induction of angiogenesis. The immune microenvironment induced by Cu2þ may indirectly lead to robust osteogenic differentiation of BMSCs via the activation of Oncostation M (OSM) pathway. Lithium is able to inhibit the GSK3 expression, which is a negative regulator of the Wnt signaling pathway. Other investigations demonstrated that lithium is able to improve fracture healing by serving as an agonist of Wnt/b-catenin signaling.

The Co²⁺ ion is believed to induce the formation of hypoxia cascade, with which stabilizing HIF-1a. Then, the cells will compensate this hypoxic environment by expressing genes (such as VEGF and EPO) that promote neovascularization and angiogenesis.

Such bioinorganic minerals can provide a bioactive coating that enhances interaction with the host tissue, improve hydrophilicity and is more favorable to remodel into native tissue. Submicron surface topography harnesses macrophage polarization - facilities binding and proliferation of macrophages, followed by M2 polzarization (M2 macrophages remove debris from sites of bone repair, restore ECM by promoting secretion of collagen from osteoblasts, and recruit MSCs). Submicron surface topography elicits endogenous cellular and biochemical response (increased levels of OPG, TGF-B1, VEGF, FGF-2, and Ang-1. Elevated levels of bone mineral content promote radiopacity of graft for imaging.

Low pH promotes better preservation of non-collagenous proteins (e.g., growth factors, exosomes, etc.). Low pH promotes extrudability/injectability of graft by allowing a layer of water around the molecules which may act as a plasticizer. By varying the ratio of precipitated bioinorganic minerals, grafts having different handling and structure can be prepared.

It will be understood that the embodiments of the present invention described hereinabove are merely exemplary and that a person skilled in the art may make variations and modifications without departing from the spirit and scope of the invention. All such variations and modifications are intended to be included within the scope of the present invention.

### EXAMPLES

### EXAMPLE 1.0 - DONOR A

### OBJECTIVE(S):

- *Investigate effects of centrifugation, lyo-protectant, pH, chemical choice, and extraction method*
- *Test for bone morphogenic (BMA) proteins*

**SAMPLE GUIDE:**

| SAMPLE ID | DESCRIPTION |
|---|---|
| AFn(4-6) | EXPERIMENTAL FIBERS - NAOH PROCESS @ PH 4,5,6 |
| AFe(4-6) | EXPERIMENTAL FIBERS - ETOH PROCESS @ PH 4,5,6 |
| AFn5LP | AFn5 w. LYOPROTECTANT SOLUTION |
| AFn5ES | AFn5 w. ADDITIONAL CENTRIFUGE SPIN CYCLE |
| N5PO | EXPERIMENTAL PRECIPITATE (NO TISSUE) - NAOH PROCESS, PH = 5 |
| DBF | DEMINERALIZED BONE FIBER CONTROL |

### PROCESS FLOW(S):

### MILLING OF CORTICAL FIBERS (APPLIES TO ALL CONTROL AND EXPERIMENTAL FIBER PRODUCTION CONDITIONS FOR ALL EXPERIMENTS HEREAFTER DESCRIBED)

To produce demineralized cortical bone fibers DCBF, the bone sections were first shaved across the shaft of the bone using a controlled advancement rate of a lathe bit having a width approximately equal to the desired length of the bone fibers. The shaft segment was secured in a vice with a sufficient portion of the shaft protruding such that the protruding portion was shaved. On a milling machine, a straight flute end-mill was set up such that its axis was parallel with the axis of the shaft. Utilizing the required length of the of the broad edge of the lathe bit, cortical fibers were shaved off of the shaft by running the end-mill back and forth along the shaft until substantially all of the bone was shaved from the shaft. The resulting bone fibers were passed through a collected for demineralization and any cortical shards residuals separated and removed from the fibers.

### DEMINERALIZATION CONTROL CONDITIONS

- Demineralized cortical bone fibers (DBF) processed as follows:
   1. Added a quantity of cortical fibers to a Caframo mixing bowl
   2. Added 0.6N HCl to the Caframo bowl (25 mL / g cortical fibers)
   3. Set Caframo to 200 rpm and allowed to soak for 15 to 20 minutes
   4. Poured the HCl and tissue out over a 212 um sieve
   5. Rinsed the tissue with DI water for 2 minutes
   6. Added the tissue back to the Caframo bowl
   7. Filled the Caframo bowl with DI water
   8. Set Caframo to 200 rpm and allowed to soak for 15 to 25 minutes
   9. Poured the water and tissue out over a 212um sieve
   10. Added the tissue back to the Caframo bowl
   11. Added 0.1M sodium phosphate dibasic buffer to the Caframo bowl (5mL / g cortical fibers)
   12. Set the Caframo speed to 200 rpm and allowed to soak for 15 to 25 minutes
   13. Poured the buffer and tissue out over a 212 um sieve
   14. Rinsed the tissue with DI water for 2 minutes
   15. Added the tissue back to the Caframo bowl
   16. Filled the Caframo bowl with DI water
   17. Set Caframo to 200 rpm and allowed to soak for 15 to 25 minutes
   18. Poured the water and tissue out over a 212um sieve
   19. Added the tissue back to the Caframo bowl
   20. Added 1X phosphate buffered saline (PBS) to the Caframo bowl (10mL per / g cortical fibers)
   21. Allowed to soak for 5 minutes without agitation
   22. Poured the buffer and tissue out over a 212 um sieve
   23. Dried the tissue with high-speed vacuum for 10 to 15 minutes
   24. Aliquoted dried tissue into individual jars
   25. Added 0.5X PBS to each jar
   26. Lyophilized for 36 hours according to the following Lyophilization Recipe (same for all examples in which lyophilization is performed)

**Lyophilization Process / Recipe**

| **THERMAL TREATMENT** | | | | |
|---|---|---|---|---|
| Step # | Temperature (°C) | Time (min) | R/H | |
| 1 | -40 | 90 | H | |
| 2 | -40 | 90 | H | |
| 3 | -40 | 60 | H | |
| 4 | -40 | 60 | H | |
| | | | | |

| **EVACUATION STEP** | | | | |
|---|---|---|---|---|
| Freeze Temperature (°C) | Additional Freeze Time (min) | | Condenser (°C) | Vacuum (mTorr) |
| 38 | 60 | | -40 | 350 |

| **PRIMARY DRYING CYCLE** | | | | |
|---|---|---|---|---|
| Step # | Temperature (°C) | Time (min) | Vacuum (mTorr) | R/H |
| 1 | 35 | 580 | 350 | H |
| 2 | 35 | 580 | 350 | H |
| 3 | 35 | 580 | 350 | H |
| | | | | |

| **SECONDARY DRYING** | | | | |
|---|---|---|---|---|
| Secondary Setpoint (°C) | | | 55 | |
| Temperature (°C) | Time (min) | | Vacuum (mTorr) | |
| 23 | 60 | | 350 | |
| | | | | |
| | **TOTAL TIME (hrs)** | | 36.00 | |

### DEMINERALIZATION EXPERIMENTAL CONDITIONS

1. Added a quantity of cortical fibers and 0.6N HCl to a Caframo mixing bowl (20mL / g cortical fibers)
2. Soaked for 15 - 25 min @ 10 - 200 rpm
3. Drained tissue over 212 um sieve with receiving basin underneath to collect all the residual demineralization solution (RDS)
4. Allowed to drip dry for 15 minutes on sieve, over the receiving basin
5. Aliquoted 1.5 g of demineralized fibers into individual 50mL conical tubes
6. Aliquoted 20 mL of RDS into each tube with tissue
   a. Did NOT add RDS to DBF control tube
   b. Added 20 mL RDS to one empty tube (n5PO condition)
7. Added alkaline solution to all tubes except DBF control
   a. For NaOH process - added 1.0N NaOH until target pH (each of 4,5,6) is reached
   b. For EtOH process - added 1.0N NaOH until target pH (each of 4,5,6) is reached + 10mL EtOH
8. Soaked for 1 - 2 hrs on orbital shaker @ 75 rpm
9. Centrifuged all tubes @ 4000rpm for 20 min @ 4°C
10. Decanted supernatant from each tube
11. For AFn5ES only - repeated Steps 9+10
12. For AFn5LP only - added relevant lyoprotectant solution
13. Lyophilized for 36 hours according to the Lyophilization Recipe provided in Example 1.0 above

### PROTEIN EXTRACTION

1. Added 6 mL of 20U/mL Collagenase II in 20mM Tris HCl solution to each tube
2. Incubated tubes overnight @ 37 °C on rotary shaker @ 75 rpm
3. Centrifuged tubes at 4000 rpm for 20 min @ 4 °C
4. Collected supernatant and aliquoted into a new 50 mL conical tube, then placed in freezer overnight(s)
5. Removed samples from freezer and allowed samples to thaw, then prepared assay per manufacturer instructions

### RESULTS:

**Table 2 - PROTEIN PANELS**

| *RAYBIOTECH QAH-BMA-2 PANEL* | | | | | | | | | | |
|---|---|---|---|---|---|---|---|---|---|---|
| *ADJUSTED DATA (pg*/*g)* | | | | | | | | | | |
| PROTEIN | DBF | N5PO | AFn4 | AFn5 | AFn6 | AFe4 | AFe5 | AFe6 | AFn5LP | AFn5ES |
| BMP-2 | 167 | 10 | 8 | 8 | 4 | 15 | 7 | 7 | 2 | 2 |
| BMP-6 | 9,973 | 1,274 | 180 | 512 | 0 | 152 | 0 | 1,975 | 169 | 0 |
| BMP-7 | 111,759 | 11,380 | 3,408 | 38,563 | 6,458 | 9,306 | 5,175 | 10,039 | 581 | 3,492 |
| Dkk-1 | 7,916 | 54 | 37 | 999 | 0 | 800 | 386 | 989 | 0 | 0 |
| MMP-3 | 9,290 | 1,387 | 1,845 | 1,215 | 1,522 | 1,025 | 1,771 | 3,709 | 0 | 212 |
| OPG | 3,547 | 0 | 0 | 446 | 0 | 25 | 21 | 0 | 0 | 0 |
| OPN | 60,263 | 1,042 | 90,745 | 123,213 | 18,418 | 40,735 | 1,397 | 2,002 | 0 | 13,672 |
| PDGF-BB | 56 | 5 | 7 | 10 | 6 | 7 | 14 | 6 | 15 | 3 |
| TGFb3 | 791 | 1,000 | 965 | 1,506 | 644 | 1,272 | 929 | 1,453 | 61 | 121 |
| TRANCE | 29,869 | 3,839 | 3,128 | 4,252 | 2,148 | 11,139 | 3,315 | 1,108 | 8,128 | 0 |

Without wishing to be bound by theory, the following observations are offered:
- The data presented above appears to indicate that there is protein in the RDS that could be retained with the described process (compared to the DBF control process where the RDS is discarded), since protein was identified in the n5PO sample, where there was no tissue included
- The data presented above appears to indicate that centrifugation may be detrimental to protein retention
- Visual inspection appeared to indicate that AFn4 and AFe4 conditions had less protein retention potential

### EXAMPLE 1.1 - DONOR A

### OBJECTIVE(S):

- *Repeat Example 1.0 with alternative protein extraction methods*
- *Test for bone morphogenic (BMA) proteins*

**SAMPLE GUIDE:**

| SAMPLE ID | DESCRIPTION |
|---|---|
| AFn(5,6) | EXPERIMENTAL FIBERS - NAOH PROCESS @ PH 5,6 |
| AFe(5,6) | EXPERIMENTAL FIBERS - ETOH PROCESS @ PH 5,6 |
| AFn5LP | AFn5 w. LYOPROTECTANT SOLUTION |
| N5PO | EXPERIMENTAL PRECIPITATE (NO TISSUE) - NAOH PROCESS PH=5 |
| n5POsd | n5PO w. DIFFERENT EXTRACTION STEPS (see below) |
| DBF | DEMINERALIZED BONE FIBER CONTROL |

### PROCESS FLOW(S):

### DEMINERALIZATION CONTROL CONDITIONS

- Demineralized cortical bone fibers (DBF) processed as in Example 1.0 above

### DEMINERALIZATION EXPERIMENTAL CONDITIONS

- Same as Example 1.0, but without Steps 9+10 *(see above)*

### PROTEIN EXTRACTION

1. Added 6 mL of 20U/mL Collagenase II in 20mM Tris HCl solution to each tube
2. Incubated tubes overnight @ 37 °C on rotary shaker @ 75 rpm
3. Added 7.5 mL 0.6N HCl to each tube
4. Incubated tubes for 2 - 4 hrs @ ambient temperature on rotary shaker @ 75 rpm
5. Centrifuged tubes @ 4000 rpm for 20 min @ 4 °C
6. Collected supernatant and aliquoted into a new 50 mL conical tube
7. Performed buffer exchange and desalting with spin columns (except for n5POsd condition)
8. Placed all samples in freezer overnight(s)
9. Removed samples from freezer and allowed to thaw
10. For n5POsd condition only, performed Step 7
11. Prepared assay per manufacturer instructions

### RESULTS:

**Table 3 - PROTEIN PANELS**

| *RAYBIOTECH QAH-BMA-2 PANEL* | | | | | | | | |
|---|---|---|---|---|---|---|---|---|
| *ADJUSTED DATA (pg*/*g)* | | | | | | | | |
| PROTEIN | DBF | N5PO | N5POsd | AFn5 | AFn6 | AFe5 | AFe6 | AFn5LP |
| BMP-2 | 850 | 2,235 | 9,765 | 17,350 | 7,078 | 6,353 | 5,558 | 10,394 |
| BMP-6 | 9 | 8,369 | 21,498 | 42,059 | 11,963 | 10,245 | 10,617 | 32,834 |
| BMP-7 | 132,152 | 26,104 | 236,798 | 518,556 | 175,150 | 119,173 | 93,507 | 312,255 |
| Dkk-1 | 9 | 170,897 | 469,893 | 888,350 | 136,036 | 136,993 | 97,919 | 533,401 |
| MMP-3 | 9 | 109,068 | 458,975 | 1,466,509 | 369,439 | 255,734 | 246,645 | 650,832 |
| OPG | 18,309 | 11,865 | 30,435 | 85,342 | 24,732 | 17,718 | 15,543 | 52,754 |
| OPN | 1,088,543 | 27,432 | 28,219 | 615,479 | 59,521 | 46,650 | 30,166 | 307,939 |
| PDGF-BB | 7 | 81 | 441 | 1,406 | 197 | 304 | 198 | 977 |
| TGFb3 | 17,754 | 103,407 | 163,797 | 743,835 | 198,410 | 236,615 | 99,832 | 343,142 |
| TRANCE | 39,912 | 533,104 | 607,337 | 1,653,230 | 248,367 | 405,468 | 208,230 | 1,079,386 |

**Table 4**

| *EXPERIMENTAL CONDITIONS SHOWN AS MULTIPLES OF DBF CONTROL (pg*/*g)* | | | | | | | | |
|---|---|---|---|---|---|---|---|---|
| PROTEIN | DBF (pg/g) | N5PO | n5POsd | AFn5 | AFn6 | AFe5 | AFe6 | AFn5LP |
| BMP-2 | 850 | 3 | 11 | 20 | 8 | 7 | 7 | 12 |
| BMP-6 | 9 | 930 | 2,389 | 4,673 | 1,329 | 1,138 | 1,180 | 3,648 |
| BMP-7 | 132,152 | 0 | 2 | 4 | 1 | 1 | 1 | 2 |
| Dkk-1 | 9 | 18,989 | 52,210 | 98,706 | 15,115 | 15,221 | 10,880 | 59,267 |
| MMP-3 | 9 | 12,119 | 50,997 | 162,945 | 41,049 | 28,415 | 27,405 | 72,315 |
| OPG | 18,309 | 1 | 2 | 5 | 1 | 1 | 1 | 3 |
| OPN | 1,088,543 | 0 | 0 | 1 | 0 | 0 | 0 | 0 |
| PDGF-BB | 7 | 12 | 68 | 216 | 30 | 47 | 30 | 150 |
| TGFb3 | 17,754 | 6 | 9 | 42 | 11 | 13 | 6 | 19 |
| TRANCE | 39,912 | 13 | 15 | 41 | 6 | 10 | 5 | 27 |

Without wishing to be bound by theory, the following observations are offered:
- Protein extraction method performed in this Example 1.1 appeared to be more effective at protein extraction compared to Example 1.0, implies the retained protein is bound to the retained mineral, i.e., coprecipitated
- Ability of the process to retain high levels of proteins versus DBF observed for all conditions
- Data presented above appear to indicate that AFn5 has the highest potential for maximum protein retention
- Data presented above appears to indicate that performing buffer exchange and desalting prior to freezing negatively influences protein stability during storage, since n5POsd > n5PO

### Example 2.0 - Donor A

### OBJECTIVE(S):

- *Optimize the AFn5 process and assess effects of alterations*
- *Compare efficacy of an alternative extraction method*
- *Test for bone morphogenic (BMA) proteins*
- *Investigate surface deposition of retained minerals via scanning electron microscopy (SEM)*

**SAMPLE GUIDE:**

| SAMPLE ID | DESCRIPTION |
|---|---|
| AFn(5.1 - 5.3) | EXPERIMENTAL FIBERS - NaOH PROCESS pH=5 @ DIFFERENT mL/g |
| n2PO | EXPERIMENTAL PRECIPITATE (NO TISSUE) - NaOH PROCESS 7.5mL/g |
| DBF | DEMINERALIZED BONE FIBER CONTROL |

### PROCESS FLOW(S):

### DEMINERALIZATION CONTROL CONDITIONS

- Demineralized bone fibers (DBF) processed as in Example 1.0 above

### DEMINERALIZATION EXPERIMENTAL CONDITIONS

1. Added a quantity of cortical fibers and 0.6N HCl to a Caframo mixing bowl (20mL / g cortical fibers)
2. Soaked for 15 - 25 min @ 10 - 200 rpm
3. Drained tissue over 212 um sieve with receiving basin underneath to collect all the residual demineralization solution (RDS)
4. Allowed to drip dry for 15 minutes on sieve, over the receiving basin
5. Aliquoted 1.5 g of demineralized fibers into individual 50 mL conical tubes
6. Aliquoted 20 mL of RDS into each tube with tissue
   a. Did NOT add RDS to DBF control tube
   b. Added 20mL RDS to one empty tube (n5.2PO condition)
7. Added alkaline solution to all tubes except DBF control
   a. For AFn5.1, added 7.0 mL/g cortical fibers
   b. For AFn5.2, added 7.5 mL/g cortical fibers
   c. For AFn5.3, added 8.0 mL/g cortical fibers
8. Soaked for 1 - 2 hrs on orbital shaker @ 75 rpm
9. Lyophilized for 36 hours according to the Lyophilization Recipe provided in Example 1.0 above

### PROTEIN EXTRACTION METHOD 1

1. Added 6mL of 20U/mL Collagenase II in 20mM Tris HCl solution to each tube
2. Incubated tubes overnight @ 37 °C on rotary shaker @ 75 rpm
3. Added 7.5 mL 0.6N HCl to each tube
4. Incubated tubes for 2 - 4 hrs @ ambient temperature on rotary shaker @ 75 rpm
5. Centrifuged tubes @ 4000 rpm for 20 min @ 4 °C
6. Collected supernatant and aliquoted into a new 50 mL conical tube, then placed in freezer overnight(s)
7. Removed samples from freezer and allowed samples to thaw, then performed buffer exchange and desalting with spin columns
8. Prepared assay per manufacturer instructions

### PROTEIN EXTRACTION METHOD 2

1. Added 4M GuHCl to each tube @ 26 mL / g
2. Incubated tubes overnight @ 2 - 8 °C on a tube rotator (i.e., a Scientific Industries Genie SI-1101 Roto-Shake) set @ speed 5
3. Centrifuged tubes at 4000 rpm for 20 min @ 4 °C
4. Collected supernatant and aliquoted into a new 50 mL conical tube, then placed in freezer overnight(s)
5. Removed samples from freezer and allowed samples to thaw, then performed buffer exchange and desalting with spin columns
6. Prepared assay per manufacturer instructions

### SEM

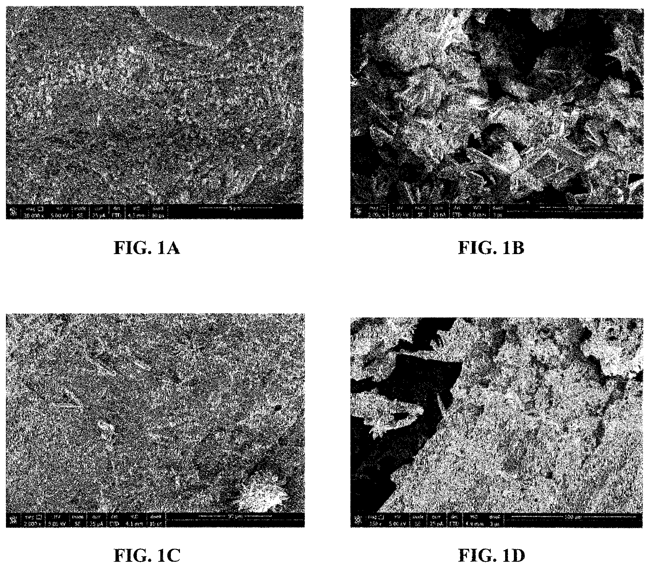

Performed by Princeton University (see FIGS. 1A - 1D)

### RESULTS:

**Table 5 - PROTEIN PANELS - RAYBIOTECH QAH-BMA-2 PANEL**

| *METHOD 1* | | | | | |
|---|---|---|---|---|---|
| *EXPERIMENTAL CONDITIONS SHOWN AS MULTIPLES OF DBF CONTROL (pg*/*g)* | | | | | |
| PROTEIN | DBF (pg/g) | n5.2PO | AFn5.1 | AFn5.2 | AFn5.3 |
| BMP-2 | 22 | 3 | 5 | 6 | 5 |
| BMP-6 | 18 | 55 | 38 | 22 | 32 |
| BMP-7 | 465 | 11 | 7 | 11 | 7 |
| Dkk-1 | 60 | 31 | 88 | 87 | 92 |
| MMP-3 | 969 | 9 | 7 | 10 | 5 |
| OPG | 575 | 2 | 4 | 4 | 4 |
| OPN | 50,383 | 1 | 3 | 2 | 1 |
| PDGF-BB | 132 | 2 | 3 | 2 | 2 |
| TGFb3 | 300 | 51 | 7 | 12 | 5 |
| TRANCE | 1,476 | 66 | 30 | 14 | 6 |

**Table 6 - BMP-2 ELISA**

| *METHOD 1 vs. METHOD 2* | | | |
|---|---|---|---|
| *Only AFn5.2 condition was tested* | | | |
| SAMPLE ID | | AVG BMP-2 (pg/g) | AVG BMP-2 (pc/g) |
| METHOD 1 | SAMPLE 1 | 59,466.00 | 54,594.11 |
| | SAMPLE 2 | 56,864.91 | |
| | SAMPLE 3 | 47,451.43 | |
| METHOD 2 | SAMPLE 1 | 64,777.83 | 66,865.13 |
| | SAMPLE 2 | 67,998.23 | |
| | SAMPLE 3 | 67,819.32 | |

### PROTEIN PANELS

Without wishing to be bound by theory, the following observations are offered:
- Ability of the process to retain high levels of proteins versus DBF observed for all conditions
- Data indicates that median of AFn5.1 + AFn5.2 has most potential for maximum protein retention
- Data indicates that protein extraction method 2 is more effective than method 1

### SEM

Without wishing to be bound by theory, the following observations are offered:
- SEM Images support hypothesis that retained mineral is redeposited on the surface of the tissue
- SEM Images show variety in both crystalline structure organization and size (note scale of images in bottom right corner) - some areas exhibit sub-micron (nano) surface roughness
- Heterogenous mineral structure is believed to be beneficial for bone healing
- Sub-micron (nano) topographies are believed to have osteoimmunomodulatory properties

### Example 3.0 - DONOR A

### OBJECTIVE(S):

- *Investigate processing of large tissue quantities*/*sample batches*
- *Investigate effect of process on cortical granules*/*particulate*
- *Test for residual calcium (RC)* + *and bone morphogenic (BMA), angiogenic (ANG), and immunomodulatory (INF) proteins*

**SAMPLE GUIDE:**

| SAMPLE ID | DESCRIPTION |
|---|---|
| AF | EXPERIMENTAL FIBERS |
| AG | EXPERIMENTAL GRANULES |
| INK | EXPERIMENTAL PRECIPITATE (NO TISSUE) (75um > X > 40um) |
| DBF | DEMINERALIZED BONE FIBER CONTROL |
| DBG | DEMINERALIZED BONE GRANULE CONTROL |
| THEORETICAL | AF + INK CONDITIONS (IF TISSUE WERE NOT SEPARATED) |

### PROCESS FLOW(S):

### MILLING OF CORTICAL GRANULES (APPLIES TO ALL CONTROL AND EXPERIMENTAL GRANULE PRODUCTION CONDITIONS FOR ALL EXPERIMENTS HEREAFTER DESCRIBED)

1. Assembled M1A or M5A Fitz Mill having a screen of size 0.065 inch (1.651 mm), per manufacturer instructions
2. Set milling speed to 4400 +/- 10 RPM
3. Added pieces of cortical bone fragments to funnel inlet
4. Stopped milling process and removed receiving tray from the mill
5. Emptied tissue over 500 um sieve with receiving pan beneath
6. Put the receiving tray back on the mill
7. Turned the mill back on and added any tissue >500 um back into the funnel inlet
8. Repeated process until all granules were < 500um

### DEMINERALIZATION CONTROL CONDITIONS

- Demineralized cortical bone fibers (DBF) processed as in Example 1.0 above
- Demineralized cortical bone granules (DBG) processed as in Example 1.0 above EXCEPT utilizing a 40 um sieve in place of the 212 um sieve

### EXPERIMENTAL CONDITIONS

1. Added bone tissue and 0.6N HCl to separate Caframo mixing bowls (20 mL / g bone tissue)
   a. a quantity of cortical fibers
   b. a quantity of cortical granules
2. Soaked for 15 - 25 min @ 10 - 200 rpm
3. Added 1.0N NaOH (7.25 mL / g bone tissue)
4. Soaked for 1 - 2 hrs @ 10 - 200 rpm
5. Transferred bone tissue to separate sieves
   a. Fibers transferred to 75 um sieve with additional 40um sieve underneath
   b. Granules transferred to 40 um sieve
6. Dried tissue in sieve via high-speed vacuum for 15 - 25 min
7. Aliquoted fibers (>75 um), granules (>40 um), and precipitate residuals (75>X>40 um) into individual lyophilizing jars
8. Lyophilized for 36 hours according to the Lyophilization Recipe provided in Example 1.0 above

### PROTEIN EXTRACTION (same as Example 2.0, Method 2)

1. Aliquoted each lyo'd sample into a 50 mL conical tube
2. Added 4M GuHCl to each tube @ 26 mL / g lyo'd sample
3. Incubated tubes overnight @ 2 - 8 °C on a tube rotator (i.e., a Scientific Industries Genie SI-1101 Roto-Shake) set @ speed 5
4. Centrifuged tubes at 4000 rpm for 20 min @ 4 °C
5. Collected supernatant and aliquot into a new 50 mL conical tube, then placed in freezer overnight(s)
6. Removed samples from freezer and allowed samples to thaw, then performed buffer exchange and desalting with spin columns
7. Prepared assay per manufacturer instructions

### RESULTS:

### RESIDUAL CALCIUM (RC) RESULTS

- *Only Experimental Fibers (AF) tested* - *5x replicates from 1 large sample*
- *Testing performed by MTF MicroLab according to OP* - *1266*

| |
|---|
| 15.47% |
| 15.21% |
| 14.74% |
| 15.28% |
| 15.82% |

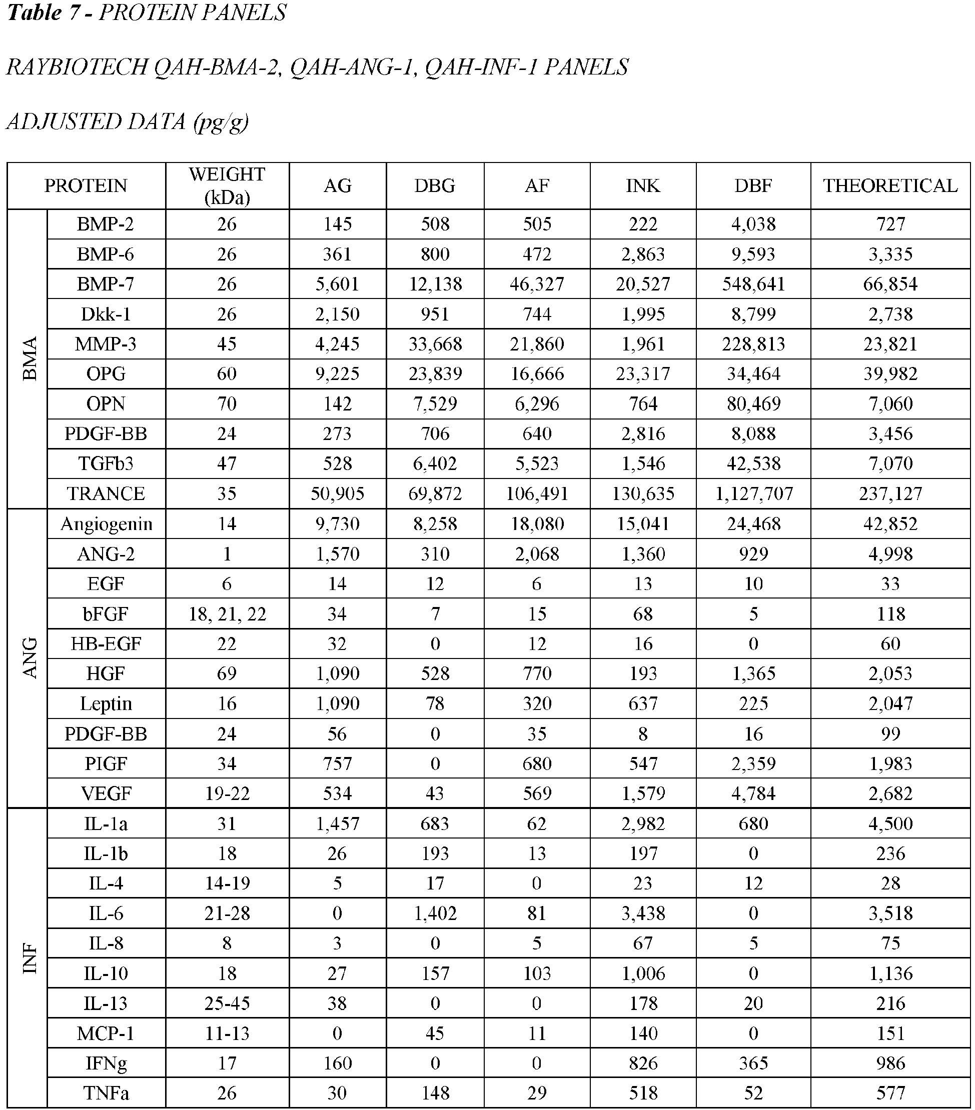

### RESIDUAL CALCIUM

Without wishing to be bound by theory, the following observations are offered:
- Experimental Fiber (AF) is not a demineralized bone matrix (DBM) based on the ASTM requirement of RC < 8% for "demineralized" bone matrix"
- With the processing described above in this Example 3.0, Calcium is likely being extracted from the matrix and redeposited on the surface of the tissue - minerals imaged based on DBM produced by the processing described in Example 2.0 are likely variations of calcium-based minerals (hydroxyapatites)
- RC levels could possibly be increased if fiber and precipitate conditions are combined (AF + INK)

### PROTEIN PANELS

Without wishing to be bound by theory, the following observations are offered:
- Significant loss in process efficacy/protein retention with scale up to large, batch processing (compared to individual sample processing as described in Examples 1.1 2.0), potential causes may be:
   ∘ Sieving + vacuuming results in loss of <40um material
   ∘ Residual demineralization solution (RDS) discarded after processing
- For ANG panel, AG > DBG, demonstrating ability of process to work on tissues other than fibers
- Potential correlation identified based on class-of-protein/type-of-substrate binding affinity by size and weight, for example:
   ∘ Largest proteins (BMA) appear to have highest affinity for fibers (AF, DBF)
   ∘ Intermediate proteins (ANG) appear to have highest affinity for granules (AG, DBG)
   ∘ Smallest proteins (INF) appear to have highest affinity for precipitate (INK)

### Example 4.0 - DONOR B

### OBJECTIVE(S):

- *Investigate fiber* + *granule combination graft*
- *Assess alternate batch processing methods to better preserve protein content*
- *Test for bone morphogenic (BMA), angiogenic (ANG), and immunomodulatory (INF) proteins*
- *Investigate phase of residual minerals in tissue via x-ray diffraction (XRD) testing*

**SAMPLE GUIDE:**

| SAMPLE ID | DESCRIPTION |
|---|---|
| AF | EXPERIMENTAL FIBERS |
| AFG | EXPERIMENTAL FIBERS + GRANULES |
| DBF | DEMINERALIZED BONE FIBER CONTROL |

### PROCESS FLOW(S):

### CONTROL CONDITIONS

- Demineralized cortical bone fibers (DBF) processed as in Example 1.0 above.

### EXPERIMENTAL CONDITIONS

1. Added bone tissues and 0.6N HCl to separate Caframo mixing bowls (20 mL / g bone tissue)
   a. a quantity of cortical fibers
   b. a quantity of cortical fibers + cortical granules
2. Soaked for 15 - 25 min @ 10 - 200 rpm
3. Added 1.0N NaOH (7.25 mL / g bone tissues (fibers and granules))
4. Soaked for 1 - 2 hr @ 10 - 200 rpm
5. Transferred bone tissues to separate, 212 um sieves with receiving basins underneath to retain RDS
6. Lightly compressed tissue and allowed to drip-dry over the basin for 10 - 15 min
7. Aliquoted relevant amount of tissue (g/cc) into individual lyophilizing jars
8. Aliquoted relevant amount of RDS (mL/cc) into each jar
9. Lyophilized for 36 hours according to the Lyophilization Recipe provided in Example 1.0 above

### PROTEIN EXTRACTION

- SAME AS Example 3.0 *(see above)*

### XRD

- Performed by Rutgers University

### RESULTS:

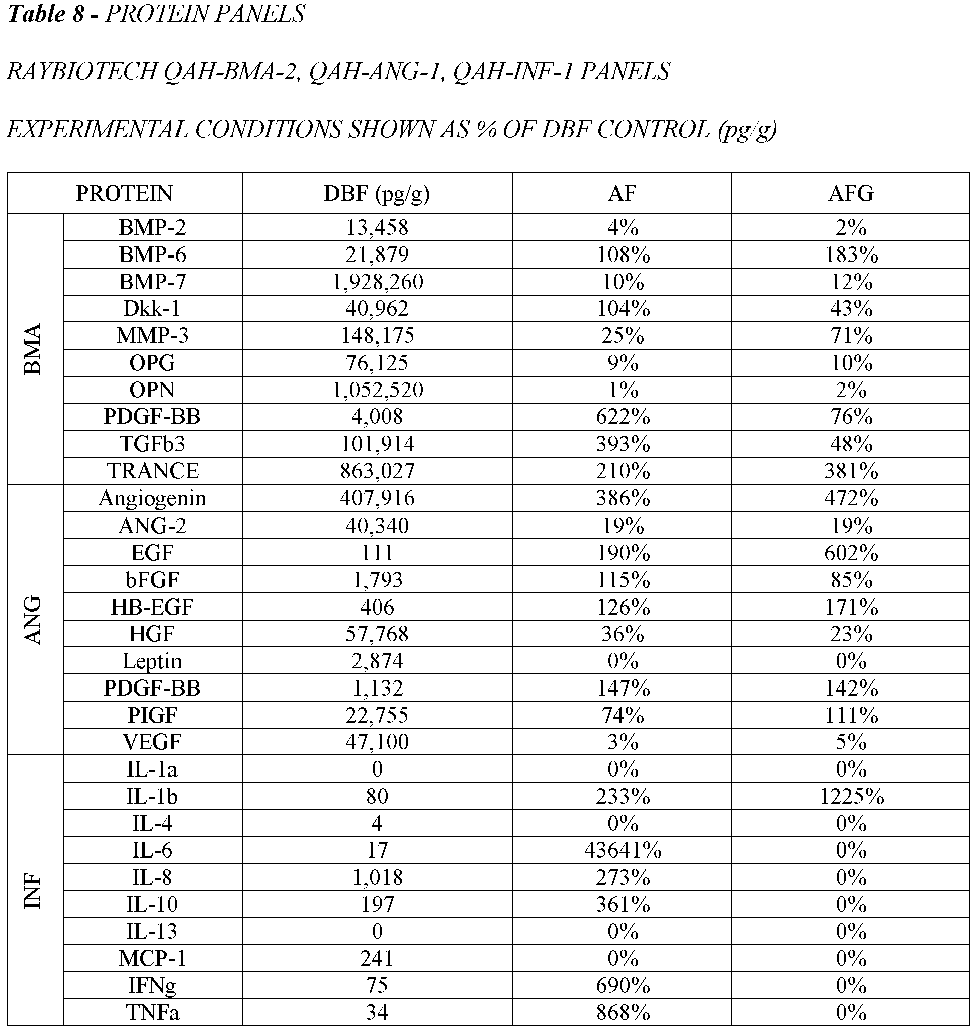

### XRD

- *Results in % of total identified mineral phase*
- *Only AFG tested*

| Halite | Ca (H P O4) (H2 0)2 | Scholzite | Fluorapatite |
|---|---|---|---|
| 7.0(6) | 9.51(12) | 1.48(3) | 82.0(6) |

### PROTEIN PANELS

Without wishing to be bound by theory, the following observations are offered:
- All conditions vary greatly across the 3 panels - there are no discernable trends
- Results achieved in this Example 4.0 appear similar those of Example 3.0 despite changes to processing methods:
   ∘ It is suspected that the extraction method utilizing GuHCl is insufficient for complete extraction of larger samples vs. Example 2.0, possibly due to common ion effect
   ∘ It is inferred that the bonds between the retained proteins and substrates are strong

### XRD

Without wishing to be bound by theory, the following observation is offered:
- Different forms of hydroxyapatite were identified - appears to further indicate surface deposition of minerals

### Example 4.1 - DONOR B

### OBJECTIVE(S):

- *Re-test conditions of Example 4. 0 (above)* + *new conditions with an alternative extraction method*
- *Test for bone morphogenic (BMA) proteins*

**SAMPLE GUIDE:**

| SAMPLE ID | DESCRIPTION |
|---|---|
| AF | EXPERIMENTAL FIBERS (same as Example 5) |
| AFG | EXPERIMENTAL FIBERS + GRANULES (same as Example 5) |
| R50 | AFG w. 50% LESS RDS |
| BT | AFG + BULK TRAY PROCESS |
| DBF | DEMINERALIZED BONE FIBER CONTROL |

### PROCESS FLOW(S):

### CONTROL CONDITIONS

- Demineralized bone fibers (DBF) processed as in Example 1.0 above

### EXPERIMENTAL CONDITIONS

- AF + AFG = SAME AS Example 4.0 *(see above)*
- R50 followed the AFG process, but the relevant mL/cc in Step 8 was reduced by 50%
- BT followed the AFG process through Step 4, then:
   1. Transferred all tissue and RDS to a large metal tray
   2. Lyophilized whole tray for 36 hours according to the Lyophilization Recipe provided in Example 1.0 above
   3. Removed resulting tissue sheet from the tray and shook off all excess excipient
   4. Transferred resulting tissue sheet to a clean tray

### PROTEIN EXTRACTION

1. Aliquoted each lyo'd sample into a 50 mL conical tube
2. Added of 20U/mL Collagenase II in 20mM Tris HCl solution to each tube @ 4 mL / g lyo'd sample
3. Incubated tubes overnight @ 37 °C on rotary shaker @ 75 rpm
4. Added 0.6N HCl to each tube @ 10 mL / g
5. Incubated tubes for 2 - 4 hrs @ ambient temperature on rotary shaker @ 75 rpm
6. Centrifuged tubes @ 4000 rpm for 20 min @ 4 °C
7. Collected supernatant and aliquoted into a new 50 mL conical tube, then placed in freezer overnight(s)
8. Removed samples from freezer and allowed samples to thaw, then perform buffer exchange and desalting with spin columns
9. Prepared assay per manufacturer instructions

### RESULTS:

**Table 9 - PROTEIN PANELS**

| *RAYBIOTECH QAH-BMA-2 PANEL* | | | | | | |
|---|---|---|---|---|---|---|
| *EXPERIMENTAL CONDITIONS SHOWN AS MULTIPLES OF DBF CONTROL (pg*/*cc)* | | | | | | |
| PROTEIN | DBF (pg/cc) | AF | R50 | BT | AFG1 | AFG2 |
| BMP-2 | 35 | 41 | 9 | 6 | 21 | 28 |
| BMP-6 | 311 | 89 | 17 | 3 | 31 | 33 |
| BMP-7 | 2763 | 63 | 17 | 7 | 21 | 35 |
| Dkk-1 | 903 | 82 | 56 | 96 | 102 | 90 |
| MMP-3 | 1893 | 41 | 9 | 3 | 11 | 10 |
| OPG | 2149 | 9 | 3 | 2 | 3 | 3 |
| OPN | 809 | 44 | 31 | 6 | 47 | 45 |
| PDGF-BB | 27 | 278 | 20 | 10 | 19 | 9 |
| TGFb3 | 3803 | 36 | 3 | 9 | 2 | 8 |
| TRANCE | 26748 | 88 | 27 | 6 | 34 | 37 |

**Table 10 - AVERAGE OF TOTAL PG/CC ACROSS ALL PROTEINS TESTED**

| ROLLUP | AVG (pg/cc) | MULT of DBF |
|---|---|---|
| DBF | 3,944 | / |
| AF | 290,251 | 73 |
| R50 | 86,445 | 21 |
| BT | 29,502 | 7 |
| AFG1 | 114,571 | 29 |
| AFG2 | 122,428 | 31 |

Without wishing to be bound by theory, the following observations are offered:
- Alternative protein extraction method performed in this Example 4.1 appears more effective at protein extraction (vs Example 4.0), from which it may be inferred that the retained protein is bound to the retained mineral
- Ability of the extraction process to retain high levels of proteins versus DBF appeared to exist for all conditions
- Based on the above data, there may be correlations between protein class/type of substrate (see Example 3.0 above), since the AF condition had the most protein content on the BMA panel
- Likely to be high levels of proteins in the RDS, since the BT condition had the second-least protein content

### Example 4.2 - DONOR B

### OBJECTIVE(S):

- *Re-test conditions of Example 4.1* + *new conditions*/*processing methods*
- *Test for and bone morphogenic (BMA), angiogenic (ANG), and immunomodulatory (INF) proteins*
- *Repeat XRD analysis*

**SAMPLE GUIDE:**

| SAMPLE ID | DESCRIPTION |
|---|---|
| AFn | EXPERIMENTAL FIBERS - NaOH PROCESS (same as Example 5) |
| AFGn | EXPERIMENTAL FIBERS + GRANULES - NaOH PROCESS (Example 5) |
| AFGc | AFG - CA(OH)2 PROCESS |
| AFGcR50 | AFGc w. 50% LESS RDS |
| DBF | DEMINERALIZED BONE FIBER CONTROL |

### PROCESS FLOW(S):

### CONTROL CONDITIONS

- Demineralized bone fibers (DBF) processed as in Example 1.0 above

### EXPERIMENTAL CONDITIONS

- AFn + AFGn = AF + AFG in Example 4.0 *(see above)*
- AFGc = AFGn but substituted 1.0N NaOH @ 7.25 mL/g for 1.0M Ca(OH)₂ @ 3 mL/g
- AFGcR50 = AFGc with 50% less RDS

### PROTEIN EXTRACTION

- SAME AS Example 4.1 *(see above)*

### XRD

- Performed by Rutgers University

### RESULTS:

**Table 11 - PROTEIN PANELS**

| *RAYBIOTECH QAH-BMA-2 PANEL* | | | | |
|---|---|---|---|---|
| *EXPERIMENTAL CONDITIONS SHOWN AS MULTIPLES OF DBF CONTROL (pg*/*cc)* | | | | |
| PROTEIN | DBF (pg/cc) | AFGn | AFGc | AFGcR50 |
| BMP-2 | 56 | 27 | 19 | 10 |
| BMP-6 | 56 | 649 | 600 | 283 |
| BMP-7 | 63671 | 3 | 3 | 2 |
| Dkk-1 | 315 | 393 | 374 | 206 |
| MMP-3 | 1468 | 59 | 35 | 24 |
| OPG | 6667 | 3 | 6 | 5 |
| OPN | 19525 | 9 | 9 | 11 |
| PDGF-BB | 36 | 43 | 326 | 73 |
| TGFb3 | 479 | 203 | 308 | 66 |
| TRANCE | 26748 | 58 | 58 | 34 |

### RAYBIOTECH QAH-ANG-1, QAH-INF-1 PANELS

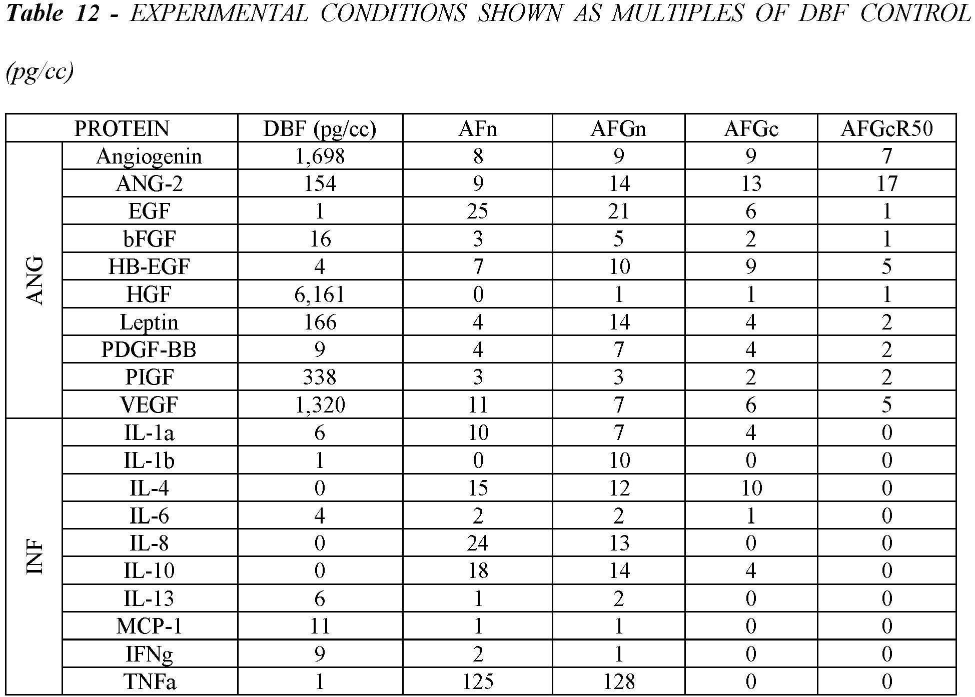

### XRD

- *Results in % of total identified mineral phase*
- *Only AFGc tested*

| Ca (H P O4) (H2 O)2 | Antarcticite | Halite | Apatite-(CaOH) | CaCl2 tetrahydrate | C6 H9 N O3 | Anhydrite |
|---|---|---|---|---|---|---|
| 53.9(2) | 14.80(10) | 1.432(17) | 5.06(6) | 8.2(3) | 10.26(17) | 6.38(5) |

### PROTEIN PANELS

Without wishing to be bound by theory, the following observations are offered:
- Calcium and sodium hydroxide [Ca(OH)₂; NaOH] appear to be comparable in retention of bone morphogenic and angiogenic proteins, although they may have higher/lower affinities for different proteins
- Sodium hydroxide appears to be more effective at retaining immunomodulatory proteins
- The above data again suggests a possible correlation between protein class/type of substrate (see Example 3.0)

### XRD

Without wishing to be bound by theory, the following observations are offered:
- Halite (sodium chloride) content reduced to approximately 1.4% without NaOH-processing
- Dicalcium phosphate- Ca(HPO4)(H2O)2 - increased to approximately 53.9% from 9.5%
- Calcium oxide identified - 5% - which is believed to be problematic for tissue stability (exothermic)

### Example 4.3 - DONOR C

### OBJECTIVE(S):

- *Test new conditions*/*processing methods*
- *Assess composition of RDS*
- *Investigate ability of tissue to retain proteins after rehydration (clinically relevant)*
- *Investigate retained minerals in tissue via atomic absorption spectroscopy*
- *Test for and bone morphogenic (BMA), angiogenic (ANG), and immunomodulatory (INF) proteins*
- *Repeat XRD analysis*

**SAMPLE GUIDE:**

| SAMPLEID | DESCRIPTION |
|---|---|
| AFGncR0 | EXPERIMENTAL FIBERS + GRANULES - COMBO PROCESS w. NO RDS |
| AFGncR50 | EXPERIMENTAL FIBERS + GRANULES - COMBO PROCESS w. 50%RDS |
| RDS1 | TOP LAYER OF RDS |
| RDS2 | BOTTOM LAYER OF RDS |
| RHD | AFGncRO + AFGncR50 - REHYDRATED PRIOR TO EXTRACTION |
| eRHD | AVG. OF AFGncR0 + AFGncR50 (EXPECTED VALUE FOR RHD) |
| DBF | DEMINERALIZED BONE FIBER CONTROL |
| MIN BONE | MINERALIZED CORTICAL BONE GRANULES |

### PROCESS FLOW(S):

### CONTROL CONDITIONS

- Demineralized cortical bone fibers (DBF) processed as in Example 1.0 above

### EXPERIMENTAL CONDITIONS

- AFGnc = AFG in Example 4.0 + 10ml Ca(OH)₂ @ Step 3 *(see above)*
- AFGncR0 = AFGnc with no RDS
- AFGncR50 = AFGc with RDS2 @ 50% of the mL/cc used in Example 4.0 above
- RDS1/2 = Top/bottom layer of the RDS after tissue drying is complete
- RHD = 1x AFGncR0 + 1x AFGncR50 combined and rehydrated with DI water, then compressed to remove all excess moisture, prior to protein extraction process
- eRHD = Estimated value for RHD based on average of (AFGncR0+AFGncR50)

### PROTEIN EXTRACTION

- SAME AS Example 4.1 *(see above)*

### ATOMIC ABSORPTION SPECTROSCOPY

- Performed by WuXi AppTec

### XRD

- Performed by Rutgers University

### RESULTS:

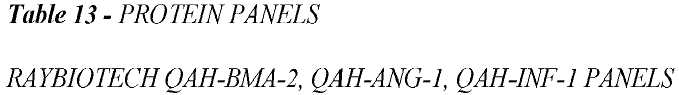

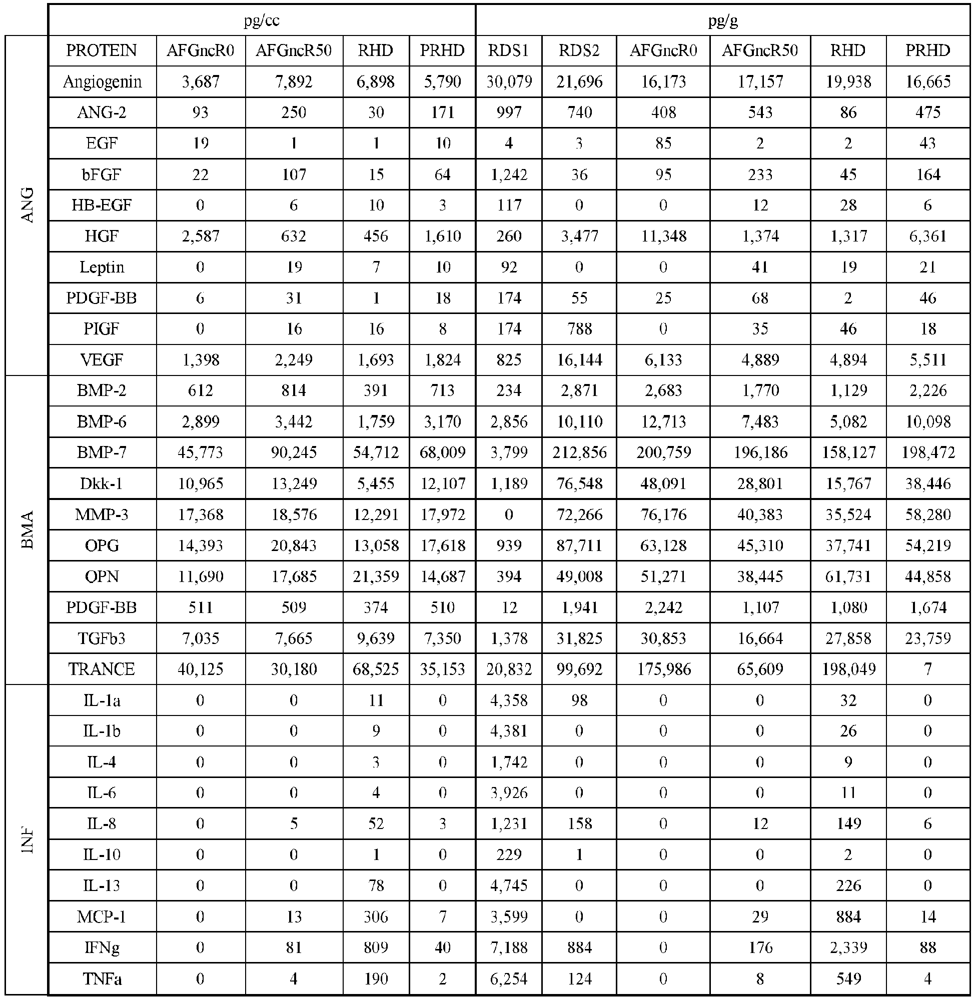

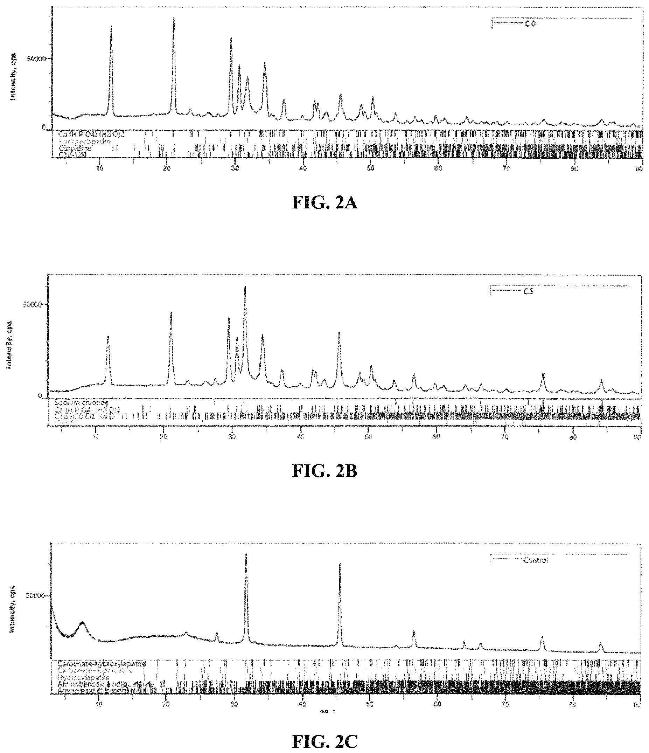

*XRD results are presented in* *FIGS. 2A-2C*

**Table 14 - ATOMIC ABSORPTION SPECTROSCOPY**

| | | | | | | | |
|---|---|---|---|---|---|---|---|
| • *Results below shown in mg of element per mg of tissue sample* | | | | | | | |
| • ^{∗}*AFGn sample from Example 4.2 Donor B, all other samples from Donor C* | | | | | | | |

| *ELEMENT* | MIN BONE | DBF | RDS I | RDS2 | AFGncR0 | AFGncR50 | AFGn^{∗} |
|---|---|---|---|---|---|---|---|
| CALCIUM | 0.313 | 0.001 | 0.137 | 0.288 | 0.237 | 0.228 | 0.244 |
| MAGNESIUM | 0.003 | 0.000 | 0.003 | 0.002 | 0.001 | 0.001 | 0.002 |
| ZINC | 0.0001 | 0.000 | 0.000 | 0.0001 | 0.0001 | 0.0001 | 0.0001 |

### PROTEIN PANELS

Without wishing to be bound by theory, the following observations are offered:
- Based on the above data, it appears that proteins are deposited / retained on the graft material in a way that prevents or minimizes their elution during rehydration of the graft material [i.e., it appears that high % (e.g., at least 90% across all test groups) of proteins is maintained compared to estimated values (RHD vs. eRHD)]
- Unexpected results for RHD on the INF panel (much higher than non-rehydrated samples)
   ∘ Potential cause: Immunomodulatory protein activation by rehydration
- Significant levels of proteins appear to have been found in the RDS:
   ∘ BMA - more found in RDS2 v RDS1
   ∘ INF - more found in RDS 1 vs RDS2
   ∘ ANG - evenly distributed between RDS1/2 overall, some proteins with specific affinities
   ∘ The above data again suggests a possible correlation between protein class/type of substrate (see Example 3.0 above)
- It appeared that AFGncR50 > AFGncR0 per cc because assumed volumes were equivalent with or without the RDS, whereas AFGncR0 > AFGncR50 per gram because AFGncR50 had additional retained mineral which greatly increases the sample's weight

### XRD

Without wishing to be bound by theory, the following observations are offered:
- AFGncR0 and AFGncR50 both have intensity peaks at a variety of wavelengths, while the control (DBF) sample showed smooth lines with intensity peaks at specific wavelengths - these results appear to indicate that the modified grafts have a heterogenous and diverse organization of mineral crystalline structures, which further supports a hypothesis that minerals are extracted from the tissue samples, retained during the process, and redeposited (e.g., coprecipitated) on the tissue's surface (see Examples 2.0, 3.0, and 4.1, above)
- Heterogenous mineral structure is suspected to be beneficial for bone healing

### ATOMIC ABSORPTION SPECTROSCOPY

Without wishing to be bound by theory, the following observations are offered:
- Graft material produced using all experimental conditions contained more calcium, magnesium, and zinc compared to DBF
- These results indicate that the process described above retains a large portion of the endogenous minerals found in mineralized bone prior to chemical processing
- Calcium, magnesium, and zinc are all known to fulfill important roles in bone healing

### EXAMPLE 5

### PROCESS 1 - COMBINING MODIFIED GRAFT AND LYOPRESERVING TECHNOLOGIES

### LYOPROTECTANT SOLUTION PREPARTION

1. Lyoprotectant solution is prepared by dissolving trehalose and epigallocatechin gallate (EGCG) into 1X PBS at concentrations of 0.3M and 2mM, respectively.
2. Following dissolution of preservative ingredients in media, solution is sterile filtered using a 0.2 µm membrane filter.

### TISSUE PREPARATION

1. Incubate viable cancellous bone granules in prepared lyoprotectant solution for up to 1 hour
2. Add cortical bone tissue(s) and 0.6N HCl to Caframo mixing bowl (20mL / g bone tissue) a. a quantity of cortical fibers and/or a quantity of cortical granules
3. Soak for 10 - 30 min @ 10 - 200 rpm
4. Add 1.0N NaOH (7.25mL/g)
5. Soak for 1 - 120 minutes @ 10 - 200 rpm
6. Transfer bone tissues to a sieve of desired pore size (e.g., 40 um, 212 um, or any suitable selected size) with receiving basins underneath to retain RDS
7. Lightly compress tissue and allow to drip-dry over the basin for 5 - 20 min
8. Aliquot relevant amount of cortical and cancellous bone tissue(s) (g/cc) into individual lyophilizing jars
9. Aliquot relevant amount of RDS (mL/cc) into each jar
10. Aliquot relevant amount of lyoprotectant solution (mL/cc) into each jar
11. Tissue is lyophilized generally according to the Lyophilization Recipe provided below

**LYOPHILIZATION RECIPE**

| **Thermal Treatment** | **Temperature (°C)** | **Time (min)** | **Vacuum (mTorr)** | **Ramp/Hold** |
|---|---|---|---|---|
| Step 1: Freeze | -30 | 120 | - | Ramp |

| **Freeze, Condenser, and Vacuum Parameters** | **Temperature (°C)** | **Time (min)** | **Vacuum (mTorr)** | **Ramp/Hold** |
|---|---|---|---|---|
| Freeze Temperature (°C) | -60 | | | Hold |
| Additional Freeze (min) | | 180 | | |
| Condenser Set Point (°C) | -60 | | | |
| Vacuum Set Point (mTorr) | | | 500 | |

| **Drying Phase Parameters** | **Temperature (°C)** | **Time (min)** | **Vacuum (mTorr)** | **Ramp/Hold** |
|---|---|---|---|---|
| Step 1 | -5 | 60 | 200 | Ramp |
| Step 2 | -5 | 320 | 200 | Hold |
| Step 3 | -2 | 10 | 200 | Ramp |
| Step 4 | -2 | 420 | 200 | Hold |
| Step 5 | 1 | 10 | 200 | Ramp |
| Step 6 | 1 | 100 | 200 | Hold |
| Step 7 | 5 | 10 | 200 | Ramp |
| Step 8 | 5 | 280 | 200 | Hold |
| Step 9 | 10 | 10 | 200 | Ramp |
| Step 10 | 10 | 345 | 200 | Hold |
| Step 11 | 14 | 10 | 200 | Ramp |
| Step 12 | 14 | 280 | 200 | Hold |

| **Secondary Drying Phase Parameters** | **Temperature (°C)** | **Time (min)** | **Vacuum (mTorr)** | **Ramp/Hold** |
|---|---|---|---|---|
| Secondary Set Point | 15 | | | |
| Post Heat Step | 15 | 5 | 100 | Hold |

### EXAMPLE 6

### PROCESS 2 COMBINING MODIFIED GRAFT AND LYOGRAFT TECHNOLOGIES

### LYOPROTECTANT SOLUTION PREPARTION

1. Lyoprotectant solution is prepared by dissolving trehalose and EGCG into 1X PBS at concentrations of 0.3M and 2mM, respectively.
2. Following dissolution of preservative ingredients in media, solution is sterile filtered using a 0.2µm membrane filter.

### TISSUE PREPARATION

1. Incubate viable cancellous bone granules in prepared lyoprotectant solution for up to 1 hour
2. Add cortical and cancellous bone tissue(s) and 0.6N HCl to Caframo mixing bowl (20mL/g)
   a. a quantity of cortical fibers and/or a quantity of cortical granules
   b. a quantity of incubated viable cancellous bone granules
3. Soak for 10 - 30 minutes @ 10 - 200 rpm
4. Add 1.0N NaOH (7.25mL/g)
5. Soak for at least 1 - 120 minute @ 10 - 200 rpm
6. Transfer bone tissues to a 212um sieve with receiving basins underneath to retain RDS
7. Lightly compress tissue and allow to drip-dry over the basin for 5 - 20 minutes
8. Aliquot relevant amount of cortical and cancellous bone tissue(s) (g/cc) into individual lyophilizing jars
9. Aliquot relevant amount of RDS (mL/cc) into each jar
10. Aliquot relevant amount of lyoprotectant solution (mL/cc) into each jar
11. Tissue is lyophilized generally according to the Lyophilization Recipe provided in Example 5 above.

### EXAMPLE 7

### PROCESS 3 COMBINING MODIFIED GRAFT AND LYOGRAFT TECHNOLOGIES

### LYOPROTECTANT SOLUTION PREPARTION

1. Lyoprotectant solution is prepared by dissolving trehalose and EGCG into 1X PBS at concentrations of 0.3M and 2mM, respectively.
2. Following dissolution of preservative ingredients in media, solution is sterile filtered using a 0.2 µm membrane filter.

### TISSUE PREPARATION

1. Add cortical and cancellous bone tissue(s) and 0.6N HCl to Caframo mixing bowl (20mL / g bone tissue)
   a. a quantity of cortical fibers and/or a quantity of cortical granules
   b. a quantity of viable cancellous bone granules
2. Soak for 10 - 30 minutes @ 10 - 200 rpm
3. Add 1.0N NaOH (7.25mL / g)
4. Soak for 1 - 120 minutes @ 10 - 200 rpm
5. Transfer bone tissues to a 212um sieve with receiving basins underneath to retain RDS
6. Lightly compress tissue and allow to drip-dry over the basin for 5 - 20 minutes
7. Aliquot relevant amount of cortical and cancellous bone tissue(s) (g/cc) into individual lyophilizing jars
8. Aliquot relevant amount of RDS (mL/cc) into each jar
9. Aliquot relevant amount of lyoprotectant solution (mL/cc) into each jar
10. Tissue is lyophilized generally according to the Lyophilization Recipe provided in example 5 above

## Claims

1. A modified tissue-derived matrix, which has been produced by processing one or more tissue samples, the matrix comprising one or more bioactive substances co-precipitated thereon, therein, or a combination thereof, wherein at least one of the one or more bioactive substances is endogenous to the one or more tissue samples.

2. The matrix of Claim 1,
wherein the one or more endogenous bioactive substances comprise one or more proteins, saccharides, lipids, minerals, organic ions, inorganic ions, inorganic substances, and combinations thereof; and/or
wherein the one or more bioactive substances are endogenous to at least one of the one or more tissue samples, and preferably further comprising one or more bioactive substances which are not endogenous to the one or more tissue samples.

3. The matrix of Claim 1 or 2,
wherein the one or more tissue samples comprise at least one tissue type selected from: adipose, amnion, placental disc, artery, bone, periosteum, cartilage, chorion, colon, dental, dermis, duodenal, endothelial, epithelial, fascia, gastrointestinal, growth plate, intervertebral disc, intestinal mucosa, intestinal serosa, ligament, liver, lung, heart, mammary, meniscal, muscle, nerve, ovarian, parenchymal organ, pericardial, periosteal, peritoneal, placental, spleen, stomach, synovial, tendon, testes, umbilical cord, urological, vascular, vein, wherein the at least one tissue type preferably comprises: adipose, amnion, bone, cartilage, chorion, dermal, fascial, intervertebral disc, ligament, meniscal, muscle, nerve, placental, tendon, umbilical cord, and combinations thereof.

4. A graft composition comprising the modified tissue-derived matrix of one of Claims 1 to 3.

5. The graft composition of Claim 4,
further comprising other materials including: natural materials, synthetic materials, metals and their alloys, ceramics, polymers, and combinations thereof; and/or
further comprising one or more additional precipitate components comprising one or more of: pharmaceutical agents, extracellular matrix compounds, antimicrobial agents, antibodies, lubricants, excipients, radiopaque compounds, contrast agents, other beneficial or useful substances, and combinations thereof.

6. A method for producing a modified tissue-derived matrix comprising one or more bioactive substances coprecipitated thereon, the method comprising:
- providing a tissue-derived substrate material by processing one or more first tissue samples by performing one or more physical techniques, one or more chemical techniques, or a combination thereof;
- providing a solution containing one or more bioactive substances;
- contacting the tissue-derived substrate material and the solution;
- depositing the one or more bioactive substances on the substrate material, in the substrate material, or both, by coprecipitating at least one of the one or more bioactive substances from the solution while in contact with at least a portion of the substrate material; and
- optionally, drying the substrate material to remove excess solution and produce a modified tissue-derived matrix.

7. The method of Claim 6,
wherein coprecipitating comprises inducing coprecipitation of at least one of the one or more bioactive substances from the solution by manipulating one or more parameters of the solution,
wherein manipulating one or more parameters of the solution preferably comprises performing one or more adjustment techniques selected from:
(a) adjusting pH of the solution by addition of a biocompatible base;
(b) adjusting concentration of one or more solvents in the solution;
(c) adding a buffered saline solution having a pH greater than the pH of the solution containing one or more bioactive substances; and
(d) adjusting concentration of one or more salts in the solution.

8. The method of Claims 6 or 7,
further comprising, prior to the step of depositing by coprecipitating, adding one or more additional precipitate components to the solution, wherein each of the one or more additional precipitate components is bioactive or not, independently of other additional precipitate components, wherein the one or more additional precipitate components preferably comprises one or more of: pharmaceutical agents, extracellular matrix compounds, antimicrobial agents, antibodies, lubricants, excipients, radiopaque compounds, contrast agents, other beneficial or useful substances, and combinations thereof; and /or
wherein the step of providing a solution containing one or more bioactive substances is intentionally or incidentally performed during the processing of the one or more first tissue samples, and wherein the solution comprises a residual process solution containing the one or more bioactive substances which are endogenous to at least one of the one or more first tissue samples, wherein the residual process solution is preferably the direct or indirect product of performing one or more processing steps on the one or more first tissue samples and selected from: cleaning, demineralizing, decellularizing, delipidizing, devitalizing, removing proteoglycans, eluting, extracting proteins, and physically agitating; and/or wherein the step of contacting the tissue-derived substrate material and the solution is intentionally or incidentally performed during the processing of the one or more first tissue samples, wherein the solution preferably comprises a residual process solution containing the one or more bioactive substances and wherein the step of contacting the tissue-derived substrate material and the residual process solution comprises: first separating the residual process solution into two or more fractions and then contacting the tissue-derived substrate material with less than all of the two or more fractions of the residual process solution, wherein a soluble fraction of the residual process solution preferably comprises soluble components of the residual process solution, and a nonsoluble fraction of the residual process solution comprises nonsoluble components of the residual process solution, and the tissue-derived substrate is contacted with either the soluble fraction or the insoluble fraction; and/or
wherein the one or more tissue samples comprise at least one tissue type selected from: adipose, amnion, placental disc, artery, bone, periosteum, cartilage, chorion, colon, dental, dermis, duodenal, endothelial, epithelial, fascia, gastrointestinal, growth plate, intervertebral disc, intestinal mucosa, intestinal serosa, ligament, liver, lung, heart, mammary, meniscal, muscle, nerve, ovarian, parenchymal organ, pericardial, periosteal, peritoneal, placental, spleen, stomach, synovial, tendon, testes, umbilical cord, urological, vascular, vein, wherein the at least one tissue type preferably comprises: adipose, amnion, bone, cartilage, chorion, dermal, fascial, intervertebral disc, ligament, meniscal, muscle, nerve, placental, tendon, umbilical cord, and combinations thereof; and/or

9. The method of one of Claims 6 to 8,
further comprising, after the step of depositing by coprecipitation, separating the solution from the tissue-derived substrate to produce the modified tissue-derived matrix comprising one or more bioactive substances coprecipitated thereon; and/or
wherein the step of providing a solution containing one or more bioactive substances comprises processing one or more second tissue samples and extracting at least one of the one or more bioactive substances from at least one of the one or more second tissue samples into a solvent comprising one or more biocompatible solvents, wherein the one or more second tissue samples preferably comprise a tissue type which is different from a tissue type of the one or more first tissue samples.

10. The method of one of Claims 6 to 9,
wherein the one or more first tissue samples comprise bone tissue which contains endogenous bioactive substances including one or more endogenous proteins and one or more endogenous minerals,
wherein processing the bone tissue comprises at least:
- reshaping the one or more bone tissue samples into at least one desired physical shape, and
- at least partially demineralizing the one or more bone samples having at least one desired physical shape by contacting them with a demineralizing solution, which produces a demineralized bone substrate material having the at least one desired physical shape, and a residual demineralizing solution having a pH and containing at least a portion of the endogenous bioactive substances,
wherein providing a solution occurs during demineralizing and the solution is the residual demineralizing solution which contains at least a portion of the endogenous bioactive substances, and
wherein coprecipitating comprises inducing coprecipitation of at least one of the one or more endogenous bioactive substances from the residual demineralizing solution by manipulating one or more parameters of the solution and allowing one or more of the endogenous proteins, endogenous minerals, or a combination thereof, to coprecipitate onto or into the demineralized bone substrate material.

11. The method of Claim 10,
wherein the one or more parameters of the residual demineralizing solution comprises the pH, and wherein the pH is adjusted to a range of from about 4 to about 6 by adding a base, while in contact with at least a portion of the demineralized first bone substrate material, wherein the base preferably comprises one or more of: sodium hydroxide, calcium hydroxide, and other biocompatible bases; and/or
wherein the at least one desired physical shape comprises a plurality of bone particles, a plurality of bone fibers, or a combination thereof.

12. The method of Claim 10 or 11,
wherein the first bone tissue sample is formed into at least a first physical shape and a second physical shape, wherein further processing and method steps are performed on the first bone tissue samples having the first physical shape and on the first bone tissue samples having the second physical shape either together, or separately.

13. The method of Claim 12,
wherein the first bone tissue sample comprises cortical bone tissue, the first physical shape comprises a plurality of cortical bone fibers, and the second physical shape comprises a plurality of cortical bone granules; and/or
wherein, when further processing and method steps are performed separately on the first bone tissue samples having the first physical shape and on the first bone tissue samples having the second physical shape, at least a portion of the first bone tissue samples having the first physical shape are combined with at least a portion of the first bone tissue samples having the second physical shape either prior to, during, or after, contacting one or both of the first bone tissue samples having the first physical shape and the first bone tissue samples having the second physical shape, with the residual demineralizing solution and coprecipitating one or more endogenous bioactive substance thereon or therein to form the modified tissue-derived matrix.

14. The method of one of Claims 10 to 13,
wherein the first bone tissue sample comprises cortical bone tissue which contains the one or more endogenous bioactive substances, wherein processing the cortical bone tissue comprises reshaping the cortical bone tissue to produce a plurality of cortical bone fibers, wherein demineralizing the plurality of cortical bone fibers produces a plurality of demineralized cortical bone fibers and the residual demineralizing solution having a pH and containing at least a portion of the endogenous bioactive substances,
wherein coprecipitating comprises inducing coprecipitation of at least one of the one or more endogenous bioactive substances from the residual demineralizing solution by manipulating one or more parameters of the solution and allowing one or more of the endogenous proteins, endogenous minerals, or a combination thereof, to coprecipitate onto, into, or both onto and into, the demineralized cancellous bone fibers to produce modified cortical bone fiber matrix;
the method further comprising:
- providing a second tissue-derived substrate material which was produced by processing one or more second tissue samples by performing one or more physical techniques, one or more chemical techniques, or a combination thereof, and wherein the one or more second tissue samples comprise a tissue type different from the one or more first tissue samples;
- combining the second tissue-derived substrate material with at least a portion of the modified cortical bone fiber matrix, either prior to, during or after coprecipitating at least one of the one or more endogenous bioactive substances from the residual demineralizing solution onto, into, or both onto and into, the demineralized cancellous bone fibers, to produce a modified mixed tissue matrix, and
- optionally, lyophilizing the modified mixed tissue matrix in the presence of a lyophilizing agent.

15. The method of Claim 14,
wherein the one or more second tissue samples comprise cancellous bone tissue,
wherein processing the one or more second tissue samples produces the second tissue-derived matrix comprising a plurality of viable cancellous bone granules; and
wherein combining the second tissue-derived substrate material with at least a portion of the modified cortical bone fiber matrix, either prior to, during or after coprecipitating at least one of the one or more endogenous bioactive substances from the residual demineralizing solution onto, into, or both onto and into, the demineralized cancellous bone fibers, produces a modified mixed cortical and cancellous bone matrix.
